# EUROPÄISCHE PATENTANMELDUNG

(11) **EP 2 821 430 A1**
(43) Veröffentlichungstag der Anmeldung: **07.01.2015**
(21) Anmeldenummer: 14171239.8
(22) Anmeldetag: 05.06.2014
(51) Int. Cl.: C08G 77/388, C08L 83/08

(54) **Siloxan-Polymere mit zentralem Polysiloxan-Polymerblock mit organofunktionellen Resten mit jeweils mindestens zwei bivalenten Gruppen ausgewählt aus Harnstoff- und/oder Carbamat-Gruppen und mindestens einem UV/Vis-Chromophor als Rest**

(30) Priorität: 01.07.2013 DE 102013106905
(71) Anmelder: Evonik Industries AG, 45128 Essen (DE)
(72) Erfinder: Ritter, Helmut, 42111 Wuppertal (DE); Knudsen, Berit, 40699 Erkrath (DE); Knott, Wilfried, 45355 Essen (DE); Henning, Frauke, 45259 Essen (DE); Hartung, Christian, 45133 Essen (DE)

(57) **Zusammenfassung**

Die Erfindung betrifft Siloxan-Polymer umfassend einen zentralen Polysiloxan-Polymerblock B mit terminalen oder am Polymerblock seitenständig gebunden organofunktionellen Resten basierend auf IPDI und UV/Vis-Chromophoren oder Derivaten, insbesondere UV-Chromophoren, die über eine hydrophobe oder hydrophile Linker-Gruppe Q1', Q2' kovalent verbunden sind sowie Zusammensetzungen enthaltend diese Siloxane. Ferner werden Verfahren zu ihrer Herstellung und ihre Verwendung offenbart.

## Beschreibung

Die Erfindung betrifft Siloxan-Polymere umfassend einen zentralen Polysiloxan-Polymerblock B mit terminalen oder am Polymerblock seitenständig gebunden organofunktionellen Resten basierend auf vorzugsweise IPDI und UV/Vis-Chromophoren oder deren Derivaten, insbesondere auf UV-Chromophoren, die über eine hydrophobe oder hydrophile Linker-Gruppe Q1 Q2' kovalent verbunden sind sowie Zusammensetzungen enthaltend diese Siloxan-Polymere. Ferner werden Verfahren zu ihrer Herstellung und ihre Verwendung offenbart.

Zur Behandlung und Modifizierung der Eigenschaften von textilen Fasern sowie keratinischen Fasern als auch der Haut kann das Eigenschaftsprofil und der erzielte Effekt im Bereich der Haar-, Körperpflege oder Behandlung von Textilien deutlich verbessert werden, indem modifizierte Siloxane als Additive zugesetzt werden. So lassen sich wesentliche Produkteigenschaften erheblich durch einen Zusatz von modifizierten Silokonen verbessern. Zu nennen sind die verbesserte Geschmeidigkeit von Cremes, das Hautgefühl, der Glanz von Haaren oder ihre Kämmbarkeit sowie auch die Wasserfestigkeit von Sonnencremes.

Es ist bekannt, dass UV-Licht der Wellenlängen von 200 bis 380 nm bzw. kurzwelliges Vis-Licht 320 bis 400 nm für das Ausbleichen und die Schädigung von Textilien, synthetischen Fasern und natürlichen Fasern (z.B. Wolle, Baumwolle und Haare) verantwortlich ist.

Um das Anwendungsspektrum der Siloxane, insbesondere Stickstoff enthaltender Siloxane, zu erweitern, besteht ein Bedarf an weiteren modifizierten Siloxanen. Ein besonderer Fokus liegt dabei auf Siloxanen, deren Gerüst gezielt Bereiche mit unterschiedlichen Eigenschaften aufweist. So besteht ein Bedarf an Siloxanen, die hydrophobe Bereiche aufweisen und zugleich Bereiche aufweisen, die hydrophil oder wasserlöslich sind. Ein besonderer Bedarf besteht an Siloxanen, die sich über Wasserstoffbrückenbindungen an natürliche Oberflächen, wie keratinische Fasern oder auch textile natürliche Fasern, anlagern können. Besonders bevorzugt sollen die Siloxane bezüglich ihrer Hydrophile und/oder Hydrophobie einstellbar sein. Bevorzugt soll ein Siloxan oder ein Gemisch von Siloxanen entwickelt werden, dass sowohl als Additiv in kosmetischen Formulierungen oder bei der Behandlung von Textilien zur Anwendung kommen kann, um die Haut, keratinische Fasern, Textilien, synthetische Fasern und natürliche Fasern vor UV-Strahlung abzuschirmen oder eine Kontrolle über den Grad der Schädigung erlauben. Es besteht daher ein wachsender Bedarf an UV-Strahlung absorbierenden Substanzen. Wünschenswert ist daher die Bereitstellung von Verbindungen, die eine Kontrolle über die UV-Strahlung ausüben können, der die Textilien bzw. die synthetischen oder natürlichen Fasern ausgesetzt werden.

Aus der Literatur ist eine große Anzahl von Verbindungen bekannt, die für den UV-Lichtschutz von Fasern, Farbstoffen und Pigmenten eingesetzt werden. Solche Verbindungen werden typischerweise direkt bei der Herstellung der Faser eingesetzt. Diese Verbindungen zeigen aber keinen pflegenden bzw. weichmachenden Effekt. Zusätzlich fehlt ihnen oft die Haftung auf der Faseroberfläche, so dass schon nach wenigen Waschvorgängen der UV-Schutz auf der Faser verlorengeht.

Es ist darüber hinaus wünschenswert, Verbindungen bereitzustellen, die einen pflegenden Effekt auf natürliche oder synthetische Fasern ausüben, eine hohe Substantivität auf der Faser zeigen und zusätzlich einen Schutz vor Schädigungen durch mechanische und/oder optische (z.B. UV-Licht) Einflüsse bieten. Um auch nach mehrmaliger Wäsche einen hinreichenden UV-Schutz bereitstellen zu können, sollten die Verbindungen auch in Weichspülformulierungen einarbeitbar sein und während des Weichspülvorgangs auf die Faser aufziehen.

Die komplexe Aufgabe der Erfindung bestand darin, modifizierte Siloxane bereitzustellen, die eine UV-Schädigung der mit den Siloxanen behandelten Oberflächen, wie Fasern oder Textilien, vermindern können und in denen der Gehalt an UV-Strahlung absorbierenden Gruppen sowie an Stickstoff enthaltenden Gruppen, insbesondere von tertiären oder quartären Stickstoff enthaltenden Gruppen und UV-Strahlung absorbierenden Gruppen unabhängig voneinander beliebig variiert werden kann. Zudem soll die Substantivität dieser Verbindungen für natürliche oder synthetische Fasern (z.B. Wolle, Baumwolle oder Haare) oder andere Oberflächen (z.B. der Haut) nach Wunsch eingestellt werden können. Durch die Siloxan-Kette soll ein glättender, pflegender und weichmachender Effekt auf natürliche oder synthetische Fasern erzielt werden. Eine weitere Aufgabe bestand darin, ein UV-Strahlung Absorbierendes Gemini-Tensid (Bistensid oder Doppel-Tensid) bereitzustellen, dass zur Anwendung im Kosmetikbereich, in der Textilindustrie, in Waschmittelformulierungen sowie als Additiv zur Beeinfluss der Oberflächeneigenschaften von Lacken, Abformmassen etc. geeignet ist und beispielsweise die Spreitung von Wassertropfen etc., positiv beeinflussen kann.

Gelöst werden die Aufgaben durch das erfindungsgemäße Siloxan und Zusammensetzungen enthaltend diese entsprechend den Merkmalen der Ansprüche 1 sowie 24 und 25 betreffend die erfindungsgemäße Zusammensetzung enthaltend mindestens ein erfindungsgemäßes Siloxan sowie durch das Verfahren zur Herstellung nach Anspruch 17 als auch die erfindungsgemäße Formulierung entsprechend den Merkmalen des Patentanspruchs 27.

Überraschend konnten die Aufgaben gelöst werden durch die Bereitstellung von Siloxan-Polymeren, die einen zentralen Polysiloxan-Polymerblock B sowie mindestens zwei terminale oder mindestens eine terminale und mindestens eine seitenkettenständige, organofunktionelle Gruppe, die jeweils abgeleitet sind aus einer Umsetzung einer Isocyanat-Gruppe von Diisocyanaten mit einem UV/Vis-Chromophor, vorzugsweise mit mindestens einem Absorptionsmaximum im Bereich von 300 bis 380 nm optional mit einem zweiten Absorptionsmaximum im Bereich von 250 bis 299 nm, bevorzugt einem UV-Chromophor, das vorzugsweise mindestens ein Absorptionsmaximum im Bereich von 280 bis 380 nm aufweist, das bevorzugt mindestens zwei Absorptionsmaxima in diesem Bereich aufweist. Besonders bevorzugt wird als UV-Chromophor Cumarin oder ein Cumarin-Derivat, wie ein Hydroxy- oder Amino-funktionelles Cumarin, Cumarin-Derivat oder deren Salz eingesetzt, wobei das gebildete UV-Chromophor-Isocyanat, insbesondere Cumarin-Isocyanat über einen Linker (Q1', Q1'AH, Q2', Q2'AH) an das Polysiloxan gebunden ist. Als Linker kommen vorzugsweise in Betracht Alkylen-, -(CH₂)ₙ- (n = 2 bis 200), Aryl-, Arylalkylen, optional mit Heterotatomen O, N und/oder S, Aminoalklyen-, quartäre Aminoalkylen, Allyl-, Ester-, Amid-, Anhydride-, Harnstoff-, (Meth)acrylat-Gruppen enthaltende Verbindungen sowie substituierte und unsubstituierte Polyether, insbesondere Polyethylenglykol-[EO]v, Propylenglykol- [PO]w oder [EO]v[PO]w.

Gegenstand der Erfindung ist mindestens ein Siloxan-Polymer der allgemeinen Formel I umfassend einen zentralen Polysiloxan-Polymerblock B,
(i.) der mit organofunktionellen Resten substituiert ist, die organofunktionellen Reste umfassen vorzugsweise einen Alkylen-Rest mit 1 bis 22 C-Atomen und/oder eine Phenyl-Gruppen oder einen Polyether,
(ii) der Polymerblock B weist lineare und/oder verzweigte Strukturen mit mindestens zwei difunktionellen Siloxan-Einheiten auf,
(iii.) der Polymerblock B weist an mindestens zwei terminalen Silizium-Atomen oder mindestens einem terminalen und mindestens einem seitenkettenständigen Silizium-Atom der Siloxan-Einheiten des Polymerblocks B die organofunktionellen Reste -Q1 und -Q2 auf, wobei die Reste gleich oder verschieden sind,

   Q2-B-Q1 (I)

   wobei -Q1 der allgemeinen Formel Ila und -Q2 der Formel Ilb entsprechen, die unabhängig gleich oder unterschiedlich sind,

   -Q1 =-Q1'-A-(C=O)-D-Q1"-A'-(C=O)-D'-Q1* (IIa)

   -Q2 = -Q2'-A-(C=O)-D-Q2"-A'-(C=O)-D'-Q2* (IIb)

   - mit A gleich -NH-, -O- oder -S- und D gleich -NH- jeweils unabhängig in Formeln IIa und Ilb,
   - mit A' gleich -NH- und D' gleich -NH-, -O- oder -S-, vorzugsweise -O- jeweils unabhängig
      in Formeln IIa und IIb, wobei jeder Rest Q1 und Q2 der Formeln IIa oder IIb mindestens zwei bivalente Gruppen, ausgewählt aus Carbamat- und Harnstoff-Gruppe aufweist, bevorzugt können IIa und IIb jeweils zwei Carbamat-Gruppen oder eine Harnstoff- und eine Carbamat-Gruppe oder zwei Harnstoff-Gruppen aufweisen,
   - mit Q1' und Q2' jeweils unabhängig umfassend einen bivalenten Kohlenwasserstoff-Rest mit 6 bis 200 C-Atomen optional umfassend mindestens ein Heteroatom O, N oder S, einen bivalenten Rest umfassend Aryl-, Arylalkyl-Gruppen optional umfassend mindestens ein Heteroatom O, N oder S oder Alkyl-, Aryl- oder Alkyl- und Aryl-Gruppen enthaltende Polyether-Reste,
   - mit Q1" und Q2" jeweils unabhängig umfassend einen bivalenten linearen, verzweigten und/oder cyclischen Alkyl-Rest mit 4 bis 200 C-Atomen, insbesondere Cyclohexenyl-Rest basiert, wie aus der Umsetzung von IPDI oder einen bivalenten Rest umfassend einen Aryl- und/oder Arylalkyl-Rest mit 6 bis 200 C-Atomen, und
   - mit -D'-Q1 und -D'-Q2* mit D', wie vorstehend definiert, wobei -D'-Q1 und -D'-Q2* jeweils unabhängig als Reste Q1* und Q2* ein UV/Vis-Chromophor als Rest umfassen, das vorzugsweise mindestens ein Absorptionsmaximum zwischen 300 und 380 nm aufweist, besonderes bevorzugt weist das Chromophor mindestens zwei Absorptionsmaxima zwischen 280 und 340 auf, die vorzugsweise zwischen 280 und 299 nm und zwischen 300 und 340 nm liegen. Vorzugsweise sind D' in beiden Resten -D'-Q1* und -D'-Q2* mit D' = -O-. Als Carbamat-Gruppen gelten sowohl -O-(C=O)-NH-als auch -S-(C=O)-NH-Gruppen, die auch als Thiocarbamate (Thiolurethan) bezeichnet werden.

Erfindungsgemäß bevorzugte Chromophore sind UV-Chromophore, bevorzugt UVA2-Chromophore, die vorzugsweise mindestens ein Absorptionsmaximum im Bereich von 320 bis 340 nm aufweisen und/oder ein UVB-Chromophor, das insbesondere ein Absorptionsmaximum im Bereich von 280-320 nm aufweist, besonderes bevorzugt weist das UVB-Chromophor zwei Absorptionsmaxima auf, wie beispielsweise zwischen 280 bis 299 nm und zwischen 300 bis 320 nm. Erfindungsgemäß bevorzugt sind Cumarin, Cumarin-Derivate oder Salze davon.

Bevorzugt entspricht B gleich Formeln IIIa oder IIIb. Vorzugsweise ist Q1' oder Q2' jeweils unabhängig ein bifunktioneller linearer, verzweigter oder cyclischer Alkylen-Rest mit 1 bis 22 C-Atomen, ein bifunktioneller Aryl-, Arylalkylen-Rest mit 6 bis 30 C-Atomen oder Polyether. Sowie vorzugsweise mit Q1" und Q2", die jeweils unabhängig bifunktionelle lineare, verzweigte oder cyclische Alkylen-Gruppe mit 1 bis 22 C-Atomen oder bifunktionelle Aryl-, Arylalkylen-Reste mit 6 bis 30 C-Atomen sind.

Im eigentlichen Sinn jedoch wird unter dem Begriff Chromophor nur der Teil einer Substanz bezeichnet, der für die Farbgebung selbst verantwortlich ist (Farbträger). Ein UV-Chromophor zeigt ein gutes Absorptionsverhalten im Spektralbereich der UV-Strahlen oder bevorzugt ein Absorptionsmaximum. Dabei nimmt das Chromophor die Energie des ultravioletten Lichts auf und verändert sich dabei vorzugsweise chemisch nicht. Die Energie wird als Wärme oder Phosphoreszenz/Fluoreszenz abgegeben. Alternativ oder zusätzlich kann das Chromophor selbst oder in seiner Umgebung molekulare Veränderungen hervorrufen. Die erfindungsgemäßen Chromophoren zeichnen sich vorzugsweise dadurch aus, dass sie die Energie als Wärme oder Phosphoreszenz/Fluoreszenz abgeben. Das bekannteste UV-Chromophor ist Melanin. Ein wichtiges UV-Chromophor ist auch die DNA selbst. Ihr Absorptionsmaximum liegt bei 260nm. Ein UV-Chromophor gemäß der Erfindung weist mindestens ein Absorptionsmaximum zwischen 280 bis 380 nm auf, bevorzugt weist es mindestens zwei Absorptionsmaxima in diesem Bereich auf, bevorzugt im Bereich von 280 bis 340, besonders bevorzugt liegt mindestens ein Maximum im Bereich von 315 bis 340 nm. Als ein UVA-Chromophor gemäß der Erfindung gilt eine Verbindung mit mindestens einem Absorptionsmaximum zwischen 315 bis 380 nm, ein bevorzugtes UVA-Chromophor gemäß der Erfindung ist ein UVA2-Chromophor mit mindestens einem Absorptionsmaximum im Bereich von 315 bis 340 nm. Als erfindungsgemäße UVB-Chromophore gelten Verbindungen mit mindestens einem Absorptionsmaximum im Bereich von 280 bis 315 nm, bevorzugte UVB-Chromophore weisen mindestens zwei Absorptionsmaxima in diesem Bereich auf. Zu den Vis-Chromophoren zählen Verbindungen mit Absorptionsmaxima von 320 bis 790, wobei als erfindungsgemäße VIS-Chromophore für einen reinen UV-Schutz nur die farblosen Vis-Chromophore mit einem Absortionsmaximum von 380 bis 400 nm zählen, während nach einer Alternative b) auch farbige Vis-Chromophore mit Absorptionsmaxima von 400 bis 790 nm verwendet werden können, wenn eine Eigenfarbe erwünscht ist. Als reiner UV-Schutz sind nur farblose Siloxan-Polymere einsetzbar, um die farbliche Erscheinung einer behandelten Oberfläche nicht zu verändern.

Entsprechend einer besonders bevorzugten Ausführungsform weisen die erfindungsgemäßen Siloxan-Polymere der allgemeinen Formeln I, IIa und IIb als -D'-Q1* und -D'-Q2* jeweils unabhängig als Reste ein UV-Chromophor umfassen mit mindestens einem Absorptionsmaximum im Bereich von 280 bis 380 nm, besonders bevorzugt mit mindestens einem Absorptionsmaximum von 300 bis 380 nm, vorzugsweise umfassen sie ein UVA2-Chromophor als Rest mit mindestens einem Absorptionsmaximum von 315 bis 340 nm, vorzugsweise von 320 bis 340 nm und/oder ein UVB-Chromophor mit mindesten einem Absorptionsmaximum im Bereich von 280-315 nm, vorzugsweise mit mindestens einem Absorptionsmaximum im Bereich von 300 bis 315 nm.

Entsprechend einer Ausführungsform umfassen die Siloxan-Polymere der allgemeinen Formeln I, IIa und IIb in -D'-Q1* und -D'-Q2* jeweils unabhängig ein Vis-Chromophor als Rest mit mindestens einem Absorptionsmaximum im Bereich von 320 bis 790 nm, insbesondere 320 bis 380 nm und/oder ein Vis-Chromophor, das zwischen 420 bis 790 nm absorbiert.

Siloxane mit dem folgenden Substitutionsmuster haben besonders vorteilhafte Eigenschaften bezüglich einem verbesserten UV-Schutz von keratinischen Fasern, insbesondere Haaren. Somit ist ein weiterer Gegenstand der Erfindung mindestens ein Siloxan-Polymer oder eine Zusammensetzung umfassend mindestens ein entsprechendes Siloxan-Polymer oder eine Mischung dieser, umfassend die Reste -Q1 und -Q2 der Formel I mit A gleich -O-, D gleich -NH-, A' gleich -NH- und D' gleich -NH- oder -O-, insbesondere mit D' gleich -O-, wobei die Reste -D'-Q1* und -D'-Q2* jeweils unabhängig abgeleitet sind aus Hydroxy-Cumarin, Hydroxy-Cumarin-Derivaten oder Amino-funktionellen Cumarin-Derivaten.

Entsprechend einer Ausführungsform umfassen die Reste -Q1 und -Q2 der Formel I die Reste -D'-Q1* und -D'-Q2*, die jeweils unabhängig abgeleitet sind aus einem Hydroxycumarin oder Hydroxycumarin-Derivat, besonders bevorzugt von 7-Hydroxycumarin (CAS:93-35-6), 4-Hydroxycumarin (CAS: 1076-38-6), 6-Hydroxycumarin (CAS: 6093-68-1) oder einem Amino-Cumarin, Amino-Cumarin-Derivat, wie 7-Amino-4-methylcoumarin (Cumarin 120), Hydroxy-Isocumarin.

Weiter bevorzugt umfassen die Reste -Q1 und -Q2 der Formel I für A gleich -O-, D gleich -NH-, A' gleich -NH- und D' gleich -O-, und die Reste -D'-Q1* und -D'-Q2* sind jeweils unabhängig abgeleitet aus einem Hydroxycumarin oder Hydroxycumarin-Derivat, insbesondere 7-Hydroxycumarin, 4-Hydroxycumarin oder 6-Hydroxycumarin.

Entsprechend einer Alternative umfasst das Siloxan-Polymer der allgemeinen Formeln I, Ila und Ilb Reste, insbesondere zusätzliche Reste, die ausgewählt sind aus -D'-Q1* und -D'-Q2* mit D' gleich -NH- mit Q1* oder Q2* jeweils unabhängig einem Amino-funktionellen Kohlenwasserstoff-Rest, insbesondere mit einem tertiären oder quartären Stickstoff-Atom. Diese Reste -D'-Q2* und -D'-Q1* sind erfindungsgemäß zusätzlich zu den -O-Q2* und -O-Q1* Resten umfassend Cumarin in einem Siloxan der Formel I vorgesehen. Die Stickstoff enthaltenden Reste bewirken eine verbesserte Substantivität auf keratinischen Fasern.

In allen allgemeinen Formeln gilt, dass das folgende Zeichen 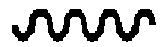 eine Bindungsstelle/ monovalente Bindungsstelle anzeigt, an die - nicht dargestellt - ein Atom, eine Gruppe bzw. Rest kovalent gebunden ist. Im Falle der substituierten Cyclohexyl-Reste basierend auf den Diisocyanatisophoron-Resten sind an den Bindungssteiien 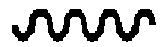 die NH(C=O)A- oder NH(C=O)D'-Reste gebunden. In den Formeln IIIa, IIIb sind die Restes Q2- und -Q1 kovalent an 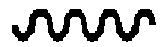 gebunden.

Entsprechend einer besonders bevorzugten Ausführungsform ist Gegenstand der Erfindung mindestens ein Siloxan-Polymer der allgemeinen Formel I oder Mischungen dieser, in dem der Polymer-Block B mindestens einer der allgemeinen Formeln IIIa oder IIIb entspricht, mit B gleich 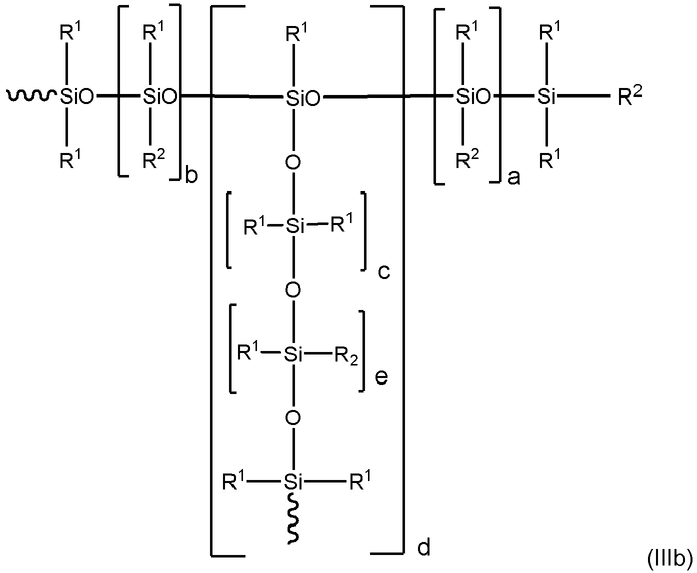 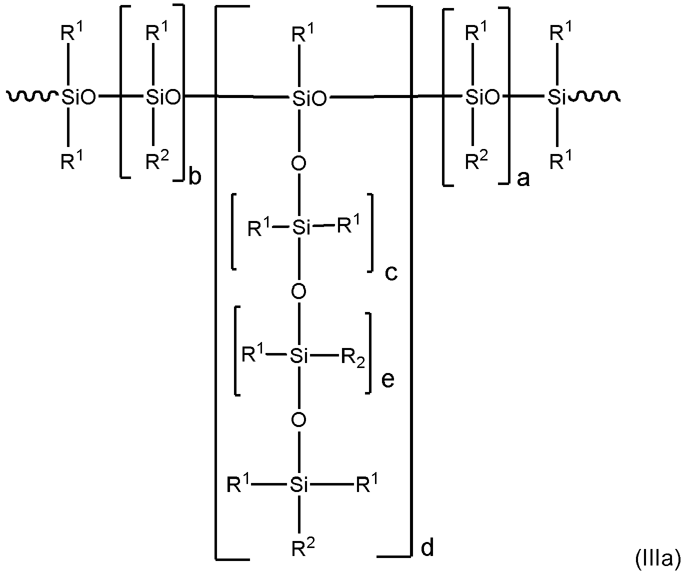 mit a, b, c, d und e in Formeln IIIa und IIIb jeweils unabhängig eine ganze Zahl mit a von 1 bis 200, insbesondere 2 bis 150, bevorzugt 2 bis 100, besonders bevorzugt 5 bis 100, vorzugsweise 20 bis 100, mit b von 0 bis 200, insbesondere 2 bis 150, bevorzugt 2 bis 100, besonders bevorzugt 5 bis 100, vorzugsweise 20 bis 100,
mit c von 0 bis 200, insbesondere 2 bis 150, bevorzugt 2 bis 100, besonders bevorzugt 5 bis 50, vorzugsweise 5 bis 20,
mit d von 0 bis 200, insbesondere 2 bis 150, bevorzugt 2 bis 100, besonders bevorzugt 5 bis 100, vorzugsweise 20 bis 100,
mit e von 0 bis 200, insbesondere 2 bis 150, bevorzugt 2 bis 100, besonders bevorzugt 5 bis 50, vorzugsweise 5 bis 20, wobei (a + b +c +d +e ) größer gleich 1, vorzugsweise größer gleich 20 sind, und mit R¹ in Formel IIIa oder IIIb jeweils unabhängig gleich oder verschieden, wobei R¹ umfasst Alkyl-Reste mit 1 bis 22 C-Atomen, vorzugsweise 1 bis 4 C-Atomen, besonders bevorzugt 1, 2, 3, 4, 6 C-Atome oder Phenyl-Reste, mit R² in Formel IIIa oder IIIb gleich Alkyl-Rest mit 1 bis 22 C-Atomen, vorzugsweise 1 bis 4 C-Atomen, besonders bevorzugt 1, 2, 3, 4, 6 C-Atome, ein Alkyl-Rest mit mindestens einem Heteroatom ausgewählt aus N, O S, wie ein Alkylamin, Alkylcarbonsäure, Alkylcarbonsäureamid, Alkylcarbonsäureanhydrid, (Meth)acrylat,Phenyl-Rest oder ein Rest -Q1'-A-(C=O)-D-Q1"-NH₂ und/oder Q2'-A-(C=O)-D-Q2"-NH₂. Besonders bevorzugt sind R¹ und R² ausgewählt aus Alkyl-Gruppen mit 1, 2, 3 oder 4 C-Atomen, insbesondere aus Methyl-Gruppen oder mindestens ein R² eine Aminoalkyl-Gruppe, insbesondere mit einer primären Amino-Gruppe oder einer quartäre Alkylamin-Gruppe.

In besonders bevorzugten Siloxan-Polymeren der Formel I, IIIa, und/oder IIIb sind die Indices b, c, d und e gleich 0 und a gleich 2 bis 200, insbesondere 2 bis 100, vorzugsweise 20 bis 100, insbesondere ist a 30 oder 80 mit einer Schwankungsbreite von plus/minus 5.

Erfindungsgemäß sind die Reste -Q1 und -Q2 in der allgemeinen Formel I unabhängig ausgewählt sind aus

-Q1 =-Q1'-A-(C=O)-D-Q1"-A'-(C=O)-D'-Q1* (IIa)

-Q2 = -Q2'-A-(C=O)-D-Q2"-A'-(C=O)-D'-Q2* (IIb)

a) mit A gleich -O-, D gleich -NH-, A' gleich -NH- und D' gleich -O-,
b) mit A gleich -O-, D gleich -NH-, A' gleich -NH- und D' gleich -NH-,
c) mit A gleich -NH-, D gleich -NH-, A' gleich -NH- und D' gleich -NH-,
d) mit A gleich -S-, D gleich -NH-, mit A' gleich -NH- und D' gleich -NH-,
e) mit A gleich -NH-, D gleich -NH-, A' gleich -NH- und D' gleich -O- oder
f) mit A gleich -S-, D gleich -NH-, A' gleich -NH- und D' gleich -O-,
g) mit A gleich -O-, D gleich -NH-, A' gleich -NH- und D' gleich -S-,
h) mit A gleich -NH-, D gleich -NH-, A' gleich -NH- und D' gleich -S- oder
i) mit A gleich -S-, D gleich -NH-, A' gleich -NH- und D' gleich -S-,
   wobei a) mit A gleich -O-, D gleich -NH-, A' gleich -NH- und D' gleich -O- besonders bevorzugt ist.

Als besonders bevorzugte Diisocyanate und daraus abgeleitete Urethane haben sich in den Resten -Q1 und -Q2 der Formel I die bivalenten Reste -Q1 "- und -Q2"- gezeigt, die unabhängig ausgewählt sind aus bivalenten, linearen, verzweigten oder cyclischen Alkylen-Resten mit 4 bis 25 C-Atomen, insbesondere mit 6, 7, 8, 9, 10, 11, 12, 13 oder 14 C-Atomen, vorzugsweise Hexylen (-CH₂)₆, Heptylen, bivalentes 2,4-Toluolyl, Diphenylmethan, polymeres Diphenylmethan, 3,5,5-Trimethyl-1-methylen-3-ethylen-cyclohexan abgeleitet aus der Umsetzung von IPDI oder 4,4'-Dicyclohexylen. Erfindungsgemäß besonders bevorzugt werden Isophorondiisocyanate (IPID) eingesetzt, die aufgrund der Strukturisomerie eine gute Kontrolle des Verfahrens erlauben, da eine Isocyanat-Gruppe reaktiver ist und somit die Bildung von hochmolekularen Polymeren vermieden werden kann. Daher ist das Verfahren sehr gut reproduzierbar und die Siloxane sind mit definierten Molekulargewichten erhältlich.

Siloxan-Polymere entsprechend der Erfindung können vorzugsweise in den Resten -Q1 und -Q2 der Formel (I) mindestens als einen der bivalenten Reste -Q1 "- und -Q2"-unabhängig einen bivalenten Cyclohexan enthaltender Rest, ausgewählt aus den Formeln Va und Vb 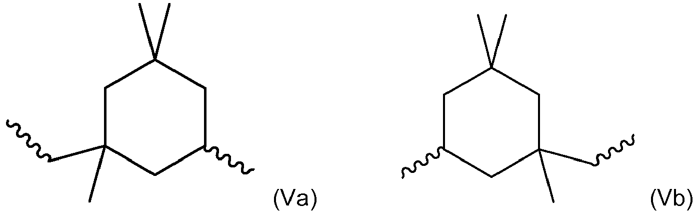 umfassen, insbesondere ist Q2"- ein bivalenter Cyclohexan enthaltender Rest der Formel Va und -Q1" der Formel Vb. Dabei wird aufgrund der unterschiedlichen Reaktivität der Isocyanat-Gruppen das Siloxan-Polymer, insbesondere der Formel I, vorzugsweise mit der dargestellten Struktur, insbesondere der Formeln Ia, Ib, Ia*, Ib*, als Hauptprodukt nach dem erfindungsgemäßen Verfahren nach Variante a) gebildet, die Verfahrensvariante b) führt bevorzugt zur Bildung der Siloxan-Polymere der Formel Ic.

Der Linker (-Q1'-A-, -Q2'-A-) ist bevorzugt aus einem olefinischen Alkohol mit 3 bis 200 C-Atomen, vorzugsweise mit 3 bis 25 C-Atomen, optional mit mindestens einem Heteroatom umfassend N, O oder S. Ebenso bevorzugt sind in den Resten -Q1 und - Q2 der Formel I die bivalenten Reste -Q1'- und -Q2'-, ausgewählt aus Alkylen-Resten mit 3 bis 22 C-Atomen optional mit mindestens einem Heteroatom umfassend N, O oder S, insbesondere -(CH₂)ₙ mit n von 3 bis 22 optional mit mindestens einem Heteroatom umfassend N, O oder S, bevorzugt sind auch Hexylen (-CH₂)₆-, Heptylen (-CH₂)₇-, Octylen (-CH₂)₈-, Nonylen (-CH₂)₉-, Decylen (-CH₂)₁₀-, Undecylen (-CH₂)₁₁-, Dodecylen (-CH₂)₁₂-, oder mit mindestens einem Heteroatom, wie Alkylen-CO-, basierend auf der Umsetzung von 10-Undecensäure, 3-Butensäure, Acrylsäure, Methacrylsäure und 5-Hexensäure, , Alkylen-O(CO)-Alkylen, Alkylen-(CO)O-Alkylen, Alkylen-NH(CO)-Alkylen, Alkylen-(CO)NH-Alkylen, Alkylen-NH(CO)NH-Alkylen, Alkylen-NH(CO)O-Alkylen, oder aus Alkyl-, Aryl- oder Alkyl- und Aryl-Gruppen enthaltenden Polyether-Resten der Formeln IVa oder IVb mit Q1' und Q2' jeweils unabhängig gleich

-T-O-(CH₂-CH₂-O-)ₓ-(CH₂-CH(R^{#})O-)_{y}-(SO)-R" (IVa)

-T-O-(CH₂-CH₂-O-)ₓ-(CH₂-CH(R^{#})O-)_{y}-R" (IVb),

mit T = bivalenter Kohlenwasserstoffrest mit 2 bis 4 C-Atomen, mit x = 0 bis 200, insbesondere mit 0 bis 100, vorzugsweise mit 0 bis 50, y = 0 bis 200, insbesondere mit 0 bis 100, vorzugsweise mit 0 bis 50, wobei x und y ganze Zahlen sind mit der Maßgabe, dass x oder y mindestens 1 ist, mit R^{#} Wasserstoff oder Methyl, R" Wasserstoff oder Alkylen, insbesondere -(CH₂)₂-, -(CH(CH₃))CH₂-, Methylen, Polymethylen oder -(CH₂)₃-, insbesondere -CH₂-CH₂-, vorzugsweise Ethylen, vorzugsweise mit T = -(CH₂)₂- oder -(CH₂)₃- . Mit R" Wasserstoff in einem Edukt oder Zwischenprodukt und lineares oder verzweigtes Alkylen in einem Zwischen- oder Endprodukt.

Besonders bevorzugte bivalenten Reste -Q1 "- und -Q2"- sind Isophoron-Derivate, Cyclohexylen enthaltende Reste und Polymethylen, wie Hexamethylen.

Nachfolgende Siloxan-Polymere sind besonders bevorzugte Siloxan-Polymere und sind ausgewählt aus Siloxan-Polymeren der Formel Ia und Ib 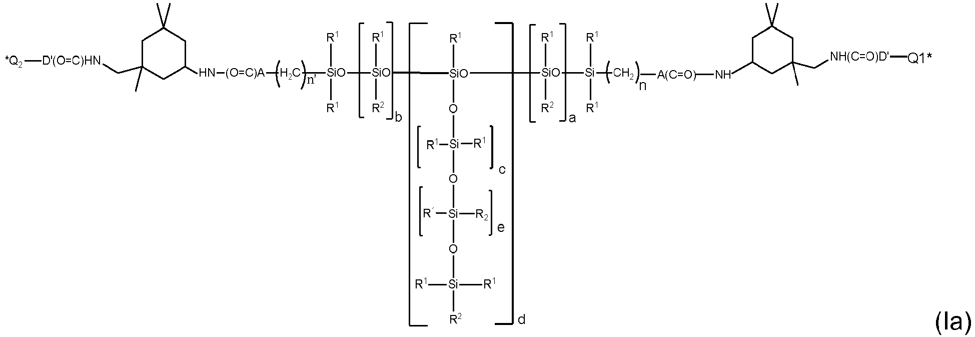 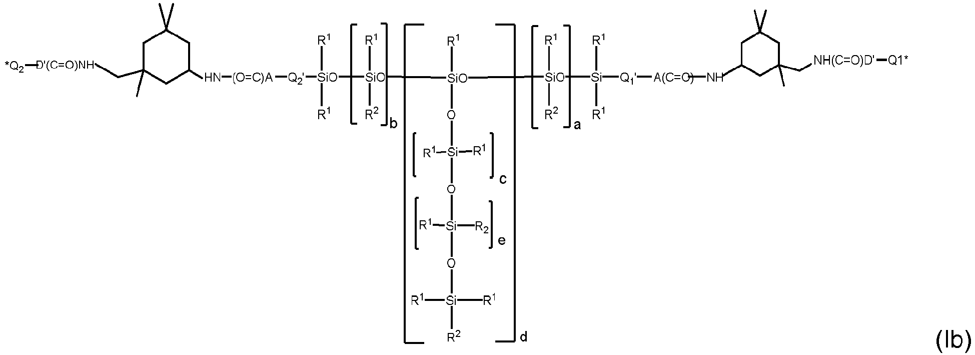 mit n oder n' jeweils unabhängig ausgewählt aus einer ganzen Zahl von 2 bis 40, insbesondere mit 3 bis 22, mit a von 1 bis 200, mit b von 0 bis 200, mit c von 0 bis 200, mit d von 0 bis 200,mit e von 0 bis 200 und mit R¹ in Formel Ia und Ib jeweils unabhängig gleich oder verschieden, wobei R¹ umfasst Alkyl-Reste mit 1 bis 4 C-Atomen oder Phenyl-Reste, mit R² gleich einem Alkyl-Rest mit 1 bis 22 C-Atomen, vorzugsweise 1 bis 4 C-Atomen, insbesondere 1, 2, 3, 4, 6 C-Atome, einem Alkyl-Rest mit mindestens einem Heteroatom ausgewählt aus N, O S, vorzugsweise Alkylamin, Glycidyloxyalkyl-Rest oder Phenyl-Rest,
insbesondere ist R² ein Alkylamin oder ein durch Reaktion eines Alkylamins mit einem Cumarin-Isocyanat erhaltener Rest, insbesondere der Formel IX, vorzugsweise der Formeln IXa, IXb, IXc und/oder IXd oder Mischungen dieser, und mit -D'-Q1* und -D'-Q2* jeweils unabhängig abgeleitet aus einem Hydroxy-Cumarin, Hydroxy-Cumarin Derivat oder deren Salzen, und in Formel Ib mit Q1' und Q2' jeweils unabhängig gleich ein lineare, cylisches, verzweigtes Alkylen mit 2 bis 40 C-Atomen oder eine Formel IVa oder IVb

-T-O-(CH₂-CH₂-O-)ₓ-(CH₂-CH(R^{#})O-)_{y}-(SO)-R" (IVa)

-T-O-(CH₂-CH₂-O-)ₓ-(CH₂-CH(R^{#})O-)_{y}-R" (IVb)

mit T = bivalenter Kohlenwasserstoffrest mit 2 bis 4 C-Atomen, mit x = 0 bis 200, insbesondere mit 0 bis 150, 1 bis 150, 5 bis 150, 5 bis 100, y = 0 bis 200, insbesondere mit 0 bis 150, 1 bis 150 5 bis 150 5 bis 100, wobei x und y ganze Zahlen sind mit der Maßgabe, dass x oder y mindestens 1 ist, mit R^{#} Wasserstoff oder Methyl, R" Wasserstoff oder Alkylen, -(CH₂)₂-, -(CH(CH₃))CH₂-, Methylen, Polymethylen oder -(CH₂)₃-, insbesondere -CH₂-CH₂-, vorzugsweise Ethylen, insbesondere mit T = -(CH₂)₂- oder - (CH₂)₃-,
R² zu einem Teil die Bedeutung der Reste R¹ haben können und die übrigen Reste R² unabhängig voneinander Reste der Formeln Id sein können, R² = -M-Z⁺ A⁻ (Ic), wobei Reste R² jeweils ein Rest der Formel -M-Z⁺ A⁻ sind, Z⁺ ein Rest der Formel Id 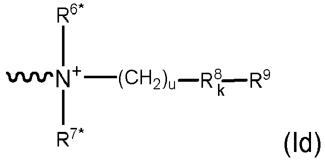 ist, R^{6*}, R^{7*} jeweils gleich oder unterschiedliche Alkylreste mit 1 bis 22 Kohlenstoffatomen oder Alkenylreste mit 2 bis 22 Kohlenstoffatomen, worin die Alkyl- oder Alkenylreste Hydroxylgruppen aufweisen können,
mit R⁸ ist -O-(C=O)- oder -NH(C=O)-,
R⁹ ein einwertiger Kohlenwasserstoffrest mit 1 bis 22 Kohlenstoffatomen sein kann, oder,
u = 0 bis 6 in Formel Id,
k = 0 oder 1 ist in Formel Id
M ein zweiwertiger Kohlenwasserstoffrest mit mindestens 4 Kohlenstoffatomen ist, der eine Hydroxylgruppe aufweise kann und der durch ein oder mehrere Sauerstoffatome unterbrochen sein kann,

A⁻ ein anorganisches oder organisches Anion, das von einer üblichen physiologisch verträglichen Säure HA herrührt, ist,

Eine Ausführungsform der Erfindung umfasst Siloxan-Polymere ausgewählt aus Siloxan-Polymeren der Formel Ia* und Ib** 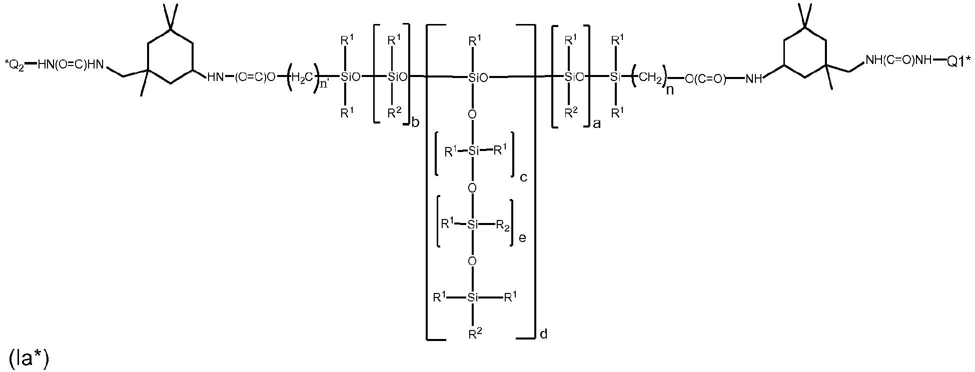 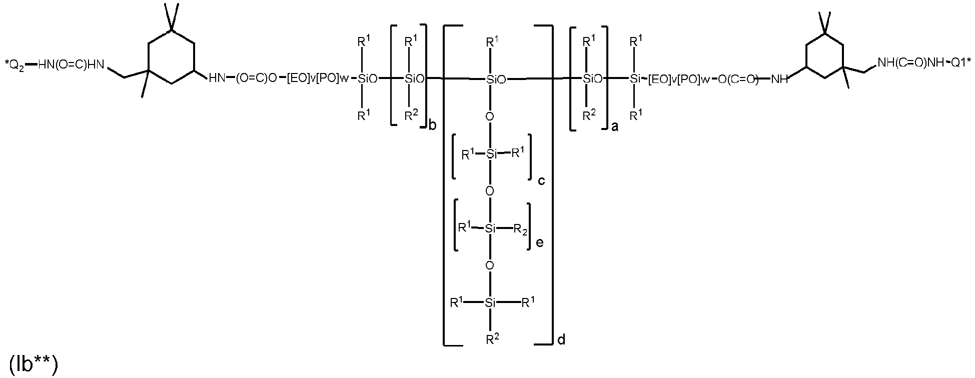 mit n oder n' jeweils unabhängig ausgewählt aus einer ganzen Zahl von 3 bis 22, mit a von 1 bis 200, insbesondere mit a von 20 bis 100, mit b von 0 bis 200, mit c von 0 bis 200, mit d von 0 bis 200, mit e von 0 bis 200, jeweils wie vorstehend definiert, und mit R¹ in Formel Ia und Ib jeweils unabhängig gleich oder verschieden, wobei R¹ umfasst Alkyl-Reste mit 1 bis 4 C-Atomen oder Phenyl-Reste, mit R² Alkyl-Rest mit 1 bis 22 C-Atomen, vorzugsweise 1 bis 4 C-Atomen, insbesondere 1, 2, 3, 4 C-Atome, einen Alkyl-Rest mit mindestens einem Heteroatom ausgewählt aus N, O S, wie Alkylamin, Glycidyloxyalkyl-Rest, oder Phenyl-Rest und mit Q1* und Q2* jeweils unabhängig Cumarin oder ein Cumarin-Derivat, insbesondere Cumarin gebunden an C-7, C-4 oder C-6 des Cumarins oder eines Cumarin-Derivates oder deren Salzen, wobei das Fragment -O-Q1* und -O-Q2* durch Reaktion der Hydroxy-Gruppe eines Cumarins mit einer Isocyanat-Gruppe die Carbamat-Gruppe bildet und in Formel Ib** mit [EO]v[PO]w, mit v von 0 bis 200 und w von 0 bis 200, insbesondere jeweils unabhängig ist mindestens v oder/und w 5 bis 100, oder gleich Q1' und Q2' jeweils unabhängig gleich Formeln IVa oder IVb,

-T-O-(CH₂-CH₂-O-)ₓ-(CH₂-CH(R^{#})O-)_{y}-(SO)-R" (IVa)

-T-O-(CH₂-CH₂-O-)ₓ-(CH₂-CH(R^{#})O-)_{y}-R" (IVb),

mit T = bivalenter Kohlenwasserstoffrest mit 2 bis 4 C-Atomen, mit x = 0 bis 200, y = 0 bis 200, vorzugsweise wie vorstehend definiert, bevorzugt ist x und/oder y 5 bis 100, wobei x und y ganze Zahlen sind mit der Maßgabe, dass x oder y mindestens 1 ist, mit R^{#} Wasserstoff oder Methyl, R" Wasserstoff oder Alkylen, -(CH₂)₂-, -(CH(CH₃))CH₂-, Methylen, Polymethylen oder -(CH) -, insbesondere -CH-CH-, und vorzugsweise Ethylen, insbesondere mit T = -(CH₂)₂- oder -(CH₂)₃-. Wobei R" in einem Edukt Wasserstoff und in einem Zwischenprodukt oder Produkt einem linearen oder verzweigten Alkylen entsprechen kann.

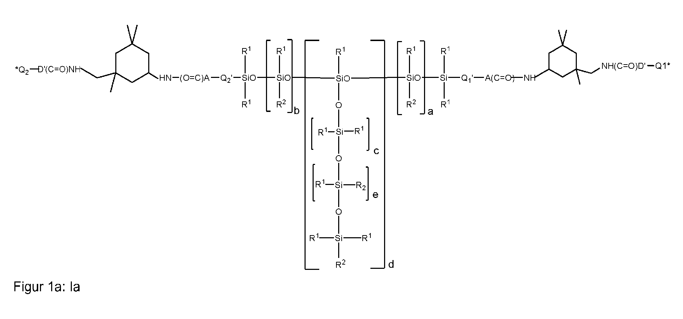

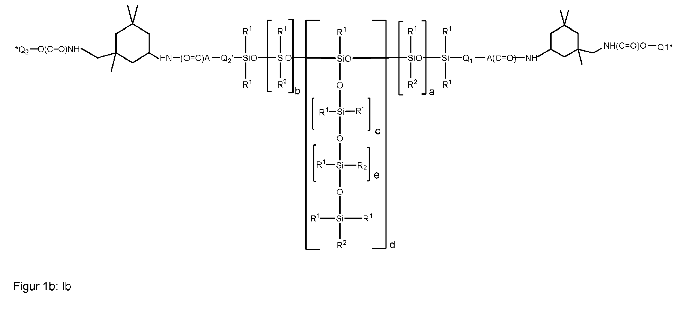

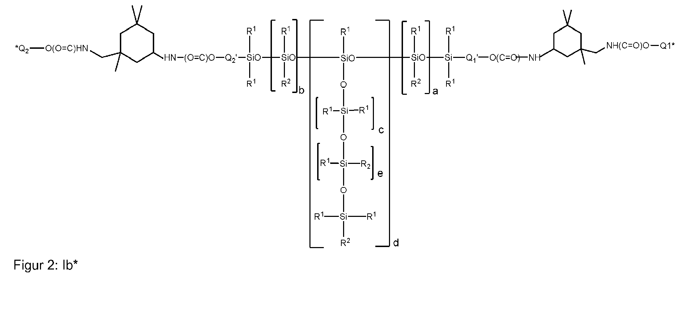

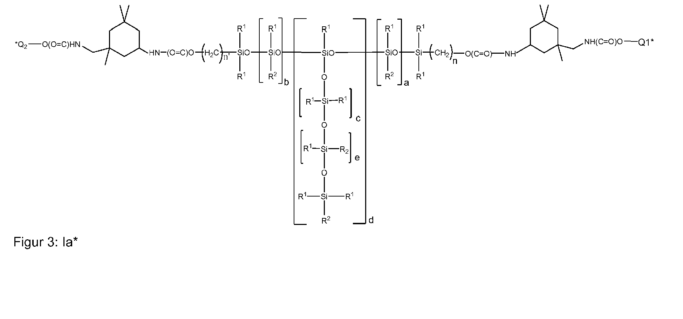

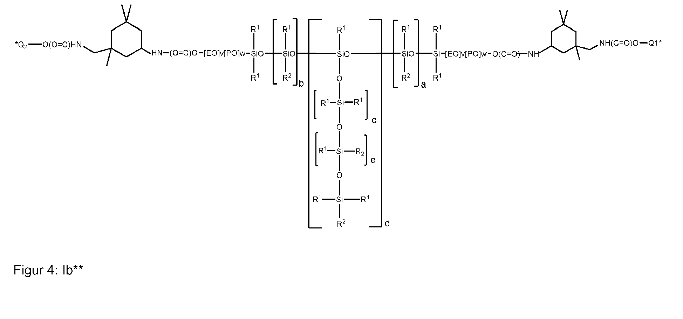

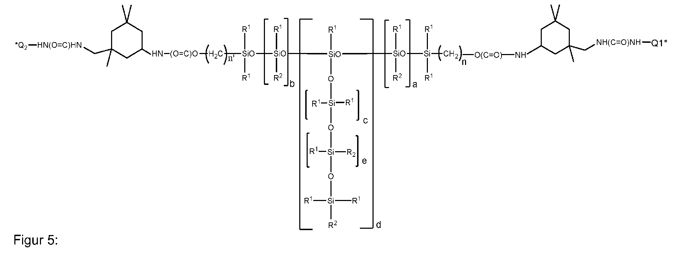

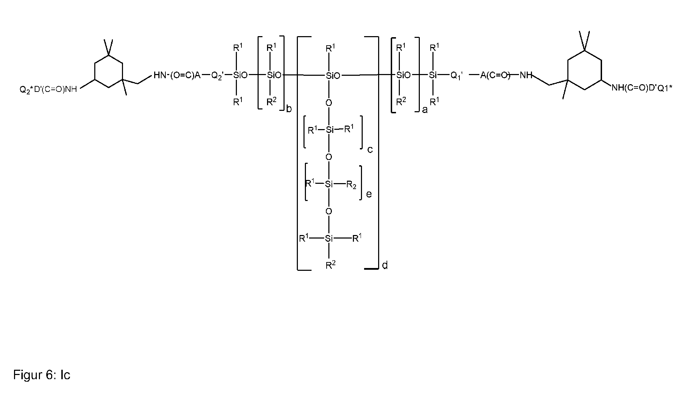

Die Figuren 1 bis 6 stellen beispielhaft die nach dem erfindungsgemäßen Verfahren erhältlichen Siloxan-Polymere der Formeln Ia, Ib, Ic dar, ohne die Erfindung auf diese Beispiele zu beschränken. Figur 1a stellt ein Siloxan-Polymer der allgemeinen Formel Ia dar, das aus der Umsetzung mit Isophorondiisocyanat und einem Hydroxy- oder Amino-funktionellen Cumarin, wie Umbelliferon erhältlich ist, der Linker ist ein Alkylen mit n oder n' jeweils unabhängig eine ganze Zahl zwischen 2 und 40, A kann -O- oder -NH-, -S- sein. Figur 1b stellt eine konkretisierte Verbindung mit Sauerstoff verbrücktem Cumarin dar, wobei die Linker Q1' und Q2' ein Alkylen oder ein Polyether sein können. Figur 2 stellt eine bevorzugte Ausführungsform der allgemeinen Formel Ib* dar, die ebenfalls durch eine Umsetzung mit einem Isophorondisocyanat und einem Hydroxy-funktionellen UV-Chromophor, wie Cumarin erhältlich ist, und die zweite Isocyanat-Gruppe des Isophorons mit einem Hydroxy-funktionalisierten Siloxan bspw. einem Hydroxyalkyl-funktionalisierten Siloxan umgesetzt wurde. Figur 3 konkretisiert in Formel Ia* die Formel Ia dahingehend, dass Q2' und Q1' jeweils ein bivalentes Alkylen sind und A gleich -O- ist. In Figur 4 wird Ib** mit einem bivalenten Polyether dargestellt -[EO]v[PO]w-, mit v und w wie vorstehend definiert. Die Siloxan-Polymere der Formeln I können auch mit einem Aminoalkyl-funktionalisierten Siloxan zu einem Siloxan-Polymer mit zwei Harnstoff-Gruppen und Cumarin-Derivaten als terminale Gruppen umgesetzt werden, s. Beispiel 3. Figur 6 zeigt ein mögliches Isomer, wenn das Verfahren nach einer alternativen Route der Variante b) über die Herstellung von UV-Chromophor-Isocyanaten, vorzugsweise Cumarin-Isocyanaten, erfolgt, indem ein Hydroxy-funktionelles Chromophor mit IPDI zu einer Verbindung der Formel IX umgesetzt wird und anschließend eine Umsetzung mit einem Siloxan-Derivat der Formel VI erfolgt (Figur 6: Ic).

Entsprechend einer besonders bevorzugten Alternative kann in dem erfindungsgemäßen Verfahren ebenfalls ein Siloxan der allgemeinen Formel XI eingesetzt werden, insbesondere wie nachstehend erläutert. In dem erfindungsgemäßen Verfahren ist R¹⁷ dann gleich Wasserstoff oder-Q1'-A-(C=O)-D-Q1"-NCO, -Q2'-A-(C=O)-D-Q2"-NCO oder-Q1'-AH, -Q2'-AH.

Ebenfalls ist ein Siloxan der allgemeinen Formel XI als erfindungsgemäßes Siloxan-Polymer, insbesondere der allgemeinen Formel I, nach dem Verfahren erhältlich, wobei R¹⁷ jeweils unabhängig gleich -Q1 und -Q2 für a2 größer gleich 1 sind, insbesondere mit a2 gleich größer gleich 2.
1) Generell ist bevorzugt mindestens ein Siloxan der allgemeinen Formel XI nach dem erfindungsgemäßen Verfahren erhältlich mit a) mit R¹⁷ wie für Siloxan-Polymere in a) definiert.
2) Ebenso kann bevorzugt mindestens ein Siloxan der allgemeinen Formel XI in dem Verfahren eingesetzt werden mit R¹⁷ entsprechend der Definition in b) für die allgemeine Formel XI

   Mₐ₁M^{A}ₐ₂M^{B}ₐ₃D_{b1}D^{A}_{b2}D^{B}_{b3}T_{c1}T^{A}_{c2}T^{B}_{c3}Q_{d1} (XI)
mit
- M: = [R¹⁶₃SiO_{1/2}]
- M^{A}: = [R¹⁷R¹⁶₂SiO_{1/2}]
- MB: = [R¹⁸R¹⁶₂SiO_{1/2}]
- D: = [R¹⁶₂SiO_{2/2}]
- D^{A}: = [R¹⁷₁R¹⁶₁SiO_{2/2}]
- D^{B}: = [R¹⁸₁R¹⁶₁SiO_{2/2}]
- T: = [R¹⁶SiO_{3/2}]
- T^{A}: = [R¹⁷SiO_{3/2}]
- T^{B}: = [R¹⁸SiO_{3/2}]
- Q: = [SiO_{4/2}],
mit
R¹⁶ unabhängig voneinander gleiche oder verschiedene lineare oder verzweigte, gesättigte oder ungesättigte Kohlenwasserstoffreste mit 1 bis 30 Kohlenstoffatomen oder auch aromatische Kohlenwasserstoffreste mit 6 bis 30 Kohlenstoffatomen bevorzugt Methyl oder Phenyl, insbesondere Methyl,
a) für das Siloxan-Polymer, insbesondere der Formel I, dargestellt über die Formel XI mit R¹⁷ ist jeweils unabhängig -Q1, -Q2 für ein Siloxan-Polymer der allgemeinen Formel I, wobei das Siloxan der Formel XI (ohne Reste R¹⁷, d.h. M^{A} = [-R¹⁶₂SiO_{1/2}], D^{A}= [-R¹⁶₁SiO_{2/2}]) und/oder T^{A} = [-SiO_{3/2}] dem Fragment B der Formel I entspricht und die Formel XI mit R¹⁷ der Formel I mit Q2-B-Q1 gleichkommt.
b) im Verfahren zur Herstellung der Siloxan-Polymere: Alternativ kann ein Siloxan der Formel XI mit R¹⁷ im Verfahren zur Herstellung mindestens eines Siloxan-Polymers, insbesondere der Formel I, eingesetzt werden, mit R¹⁷ umfassend -Q1'-AH, -Q2'-AH, -Q1'-A-(C=O)-D-Q1"-NCO, OCN-Q2"-D-'(O=C)-A-Q2'-, Wasserstoff für Si-H-Gruppe,-OH, -OR¹⁶, insbesondere -OMe, -AH, besonders bevorzugt ist in einer Alternative R¹⁷ ein gesättigter Kohlenwasserstoffrest mit endständiger -OH oder -NH₂-Gruppe, vorzugsweise mit 8 bis 30, besonders bevorzugt mit 8 bis 20 C-Atomen, insbesondere in M^{A} und optional D^{A} oder R¹⁷ gleich R¹⁸ in M^{B}, D^{B} und/oder T^{B},
R¹⁸ unabhängig voneinander gleiche oder verschiedene lineare oder verzweigte, gesättigte oder olefinisch ungesättigte Kohlenwasserstoffreste mit 8 bis 30 Kohlenstoffatomen, beispielsweise Decyl-, Dodecyl, Tetradecyl-, Hexadecyl-, Octadecyl-, insbesondere Hexadecyl- und Octadecyl-,
einen aromatischen Kohlenwasserstoffrest mit 6 bis 40 Kohlenstoffatomen, einen Alkylarylrest mit 7 bis 40 Kohlenstoffatomen,
einen durch ein oder mehrere Heteroatome (Sauerstoff, NH, NR' mit R' gleich einem gegebenenfalls Doppelbindungen enthaltenden C1 bis C30-Alkylrest, insbesondere-CH₃) unterbrochenen linearen oder verzweigten gegebenenfalls Doppelbindungen enthaltenden aliphatischen Kohlenwasserstoffrest mit 2 bis 30 Kohlenstoffatomen,
einen durch eine oder mehrere Funktionalitäten, ausgewählt aus der Gruppe -OH, -O-C(O)-, -(O)C-O-, -NH-C(O)-, -(O)C-NH, - (CH₃)N-C (O)-, -(O)C-N(CH₃)-, -S(O₂)-O-, - O-S(O₂)-, -S(O₂)-NH-, -NH-S(O₂)-, -S (O₂)-N (CH₃)-, -N(CH₃)-S(O₂)-, unterbrochenen linearen oder verzweigten gegebenenfalls Doppelbindungen enthaltenden aliphatischen Kohlenwasserstoffrest mit 2 bis 30 Kohlenstoffatomen,
einen endständig OH, OR', NH₂, N(H)R', N(R')₂ (mit R' gleich einem gegebenenfalls Doppelbindungen enthaltenden C1- bis C30-Alkylrest) funktionalisierten linearen oder verzweigten gegebenenfalls Doppelbindungen enthaltenden aliphatischen oder cycloaliphatischen Kohlenwasserstoffrest mit 1 bis 30 Kohlenstoffatomen oder
einen blockweise oder statistisch aufgebauten Polyether gemäß -(R⁵-O)ₙ-R⁶, wobei R⁵ ein 2 bis 4 Kohlenstoffatome enthaltender linearer oder verzweigter Kohlenwasserstoffrest, n 1 bis 100, vorzugsweise 2 bis 60, ist und R⁶ Wasserstoff, einen linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einen aromatischen Kohlenwasserstoffrest mit 6 bis 40 Kohlenstoffatomen, einen Alkylarylrest mit 7 bis 40 Kohlenstoffatomen,
oder ein Rest -C(O)-R⁷ mit R⁷ gleich einem linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einem aromatischen Kohlenwasserstoffrest mit 6 bis 40 Kohlenstoffatomen, einem Alkylarylrest mit 7 bis 40 Kohlenstoffatomen, besonders bevorzugt ist in einer Alternative R¹⁸ ein gesättigter Kohlenwasserstoffrest mit endständiger -NH₂-Gruppen, vorzugsweise mit 8 bis 30 C-Atomen, besonders bevorzugt mit 8 bis 20 C-Atomen,
mit
a1 = 0-200, bevorzugt 1-60, insbesondere 0,
a2 = 0-30, bevorzugt 1-20, insbesondere 2-10, wie 2, 3, 4, 5, 6, 7, 8, 9, 10
a3= 0-30, bevorzugt 1-20, insbesondere 0, wie 1, 2, 2, 3, 4, 5, 6, 7, 8, 9, 10, 15, 20
b1 = 2 bis 5000, bevorzugt 10 bis 1000, insbesondere 10-500, besonders bevorzugt 2 bis 100, vorzugsweise 10 bis 100,
b2 = 0 bis 100, bevorzugt 1 bis 30, insbesondere 1 bis10 oder 0,
b3 = 0 bis 100, bevorzugt 0 bis 30, insbesondere 1 bis 10 oder 0,
c1 = 0 bis 30, bevorzugt 1 bis 30,
c2 = 0 bis 30, bevorzugt 0 bis 5, insbesondere 0,
c3 = 0 bis 30, bevorzugt 0 bis 5, insbesondere 0,
d1 = 0 bis 30, bevorzugt 0 bis 5, vorzugsweise 0
mit der Maßgabe, dass mindestens einer der Indices ausgewählt aus a1, a2 und a3 ungleich 0 ist, insbesondere mit (a2+b2+c2) größer gleich 1, vorzugsweise ist a2 eine ganze Zahl zwischen 2 und 10, vorzugsweise 2 bis 5, wie 2, 3, 4 oder 5, wobei weiter bevorzugt ist, dass b1 von 10 bis 150, vorzugsweise 10 bis 100 ist. Optional können zusätzlich a1 und/oder a3 eine ganze Zahl zwischen 2 und 10, vorzugsweise 2 bis 5, wie 2, 3, 4 oder 5 sein. Mit der Maßgabe, dass (c1 + c2 + c3) eine ganze Zahl größer gleich 1 ist, wenn die Summe aus (a1+a2+a3) eine ganze Zahl größer 2 ist. Entsprechend einer Alternative können auch a1 und/oder a3 zusätzlich eine ganze Zahl größer 1 sein.

Erfindungsgemäß bevorzugte im Verfahren eingesetzte mindestens eine Gruppe ausgewählt aus Hydroxy- und Amino-Gruppe aufweisende Siloxane sind gekennzeichnet durch die Parameterkennzeichnung ausgewählt aus der Gruppe:
a1 = 0, a2= 2, a3 = 0, b1 = 10-100, b2 = 0, b3 = 0, c1= 0, c2 = 0, c3 = 0 und d1 = 0, a1 = 0, a2= 2, a3 = 0, b1 = 10-100, b2 = 1-30, b3 = 0, c1= 0, c2 = 0, c3 = 0 und d1 = 0;
a1 = 0, a2= 2, a3 = 0, b1 = 20 -40, b2 = 1-30, b3 = 0, c1= 0, c2 = 0, c3 = 0 und d1 = 0, a1 = 0, a2= 2, a3 = 0, b1 = 41-90, b2 = 1-30, b3 = 0, c1= 0, c2 = 0, c3 = 0 und d1 = 0, a1 = 0, a2= 2, a3 = 0, b1 = 5-350, b2 = 0, b3 = 0, c1= 0, c2 = 0, c3 = 0 und d1 = 0, a1 = 0, a2= 2, a3 = 0, b1 = 15-200, b2 = 0, b3 = 0, c1= 0, c2 = 0, c3 = 0 und d1 = 0, a1 = 0, a2= 2, a3 = 0, b1 = 10-150, b2 = 0, b3 = 1 bis 5, c1= 0, c2 = 0, c3 = 0 und d1 = 0;
a1 = 0, a2= 0, a3 = 2, b1 = 5-350, b2 = 0, b3 = 0, c1= 0, c2 = 0, c3 = 0 und d1 = 0, a1 = 0, a2= 0, a3 = 2, b1 = 15-200, b2 = 0, b3 = 0, c1= 0, c2 = 0, c3 = 0 und d1 = 0, a1 = 2, a2= 0, a3 = 2 bis 5, b1 = 10-150, b2 = 1-30, b3 = 0, c1≥ 0, c2 ≥ 0, c3 ≥ 0 und d1 = 0; mit (c1 + c2 + c3) größer gleich 1 bis 3
a1 = 0, a2= 2, a3 = 0, b1 = 10-150, b2 = 0, b3 = 1-2, c1= 0, c2 = 0, c3 = 0 und d1 = 0, a1 = 0, a2= 2, a3 = 0, b1 = 51-90, b2 = 0, b3 = 1-2, c1= 0, c2 = 0, c3 = 0 und d1 = 0, a1 = 0, a2= 0, a3 = 2, b1 = 10-50, b2 = 0, b3 = 1-2, c1= 0, c2 = 0, c3 = 0 und d1 = 0, a1 = 0, a2= 1, a3 = 1, b1 = 10-150, b2 = 0, b3 = 1 bis 5, c1 =0, c2 = 0, c3 = 0 und d1 = 0.

Die in Formeln I, II, III, IV, XI sowie allen dazugehörigen Unterstrukturen, die beispielsweise mit arabischen Buchstaben benannt sind, wiedergegebenen Indexzahlen a, b, c, d, e, f" a1, a2, a3, b1, b2, b3, c1, c2, c3, d1, d2, d3, v, w, n, n' etc. und die Wertbereiche der angegebenen Indices verstehen sich als die Mittelwerte der möglichen statistischen Verteilung der tatsächlich vorhandenen Strukturen und/oder deren Mischungen. Dies gilt auch für als solche an sich exakt wiedergegebene Strukturformeln, wie beispielsweise für Formel I, II, III und III, oder IIa, IIb, IIIa, IIIb.

Statistische Verteilungen können blockweise aufgebaut sein mit einer beliebigen Anzahl an Blöcken und einer beliebigen Sequenz oder einer randomisierten Verteilung unterliegen, sie können auch alternierend aufgebaut sein oder auch über die Kette einen Gradienten bilden, insbesondere können sie auch alle Mischformen bilden, bei denen gegebenenfalls Gruppen unterschiedlicher Verteilungen aufeinander folgen können. Spezielle Ausführungen können dazu führen, dass die statistischen Verteilungen durch die Ausführung Beschränkungen erfahren. Für alle Bereiche, die nicht von der Beschränkung betroffen sind, ändert sich die statistische Verteilung nicht.

Vorzugsweise sind ist R¹⁷ in M^{A} und/oder D^{A} optional T^{A} ausgewählt aus den beiden nachfolgenden Formeln IX1 und IX2 oder umfassen einen Rest der Formeln XIIa oder Xllb.

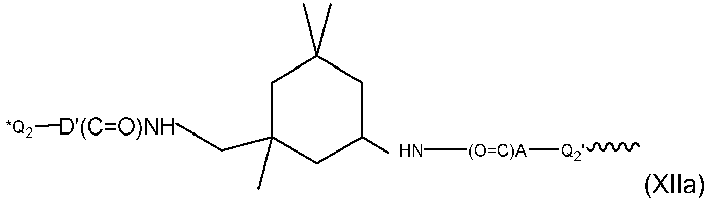

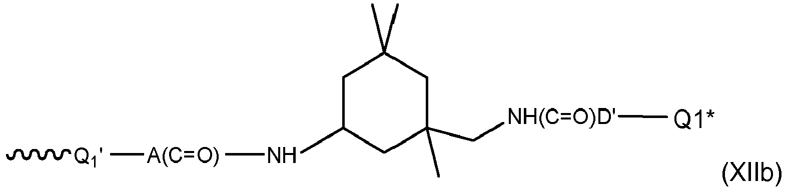

Zusätzlich zu den UV-Chromophor-Isocyanaten, wie Cumarin-Isocyanaten der allgemeinen Formeln (IXa bis IXd) können auch weitere substituierte Isocyanat-Derivate, vorzugsweise aus der Umsetzung mit Diaminen umfassend eine tertiäre und eine primäre Amino-Gruppen an Kohlenwasserstoffen optional umfassend O oder N in der Reaktion mit einem reaktiven Hydroxy- oder Amino-funktionellen Siloxan, bspw. der Formel VI umgesetzt werden. Die Umsetzung kann auch in einer Mischung umfassen Cumarin-Isocyanate erfolgen. In Formeln IX1, IX2 mit Z = -Q1*, Q2*, wie abgeleitet aus Formel XIII oder einem Amin, einem Diamin, wie DMPAPA, etc.

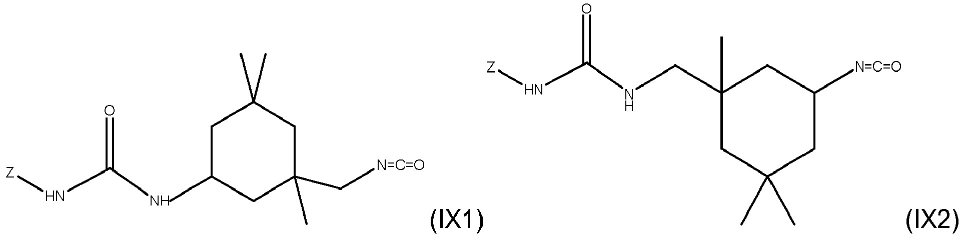

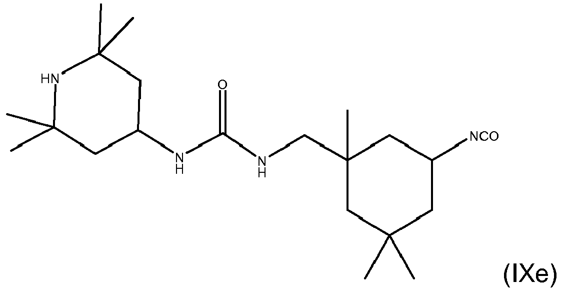

Nach einer Alternative kann zusätzlich zur Umsetzung mit Hydroxy-Cumarin oder einem Hydroxy-Cumarin-Derivat eine zusätzliche Umsetzung mit sterisch gehinderten Aminen mit einer primären Amino- oder Hydroxy-Gruppe erfolgen, besonders bevorzugt sind die Diamine mit einem sterisch gehindertem Stickstoff als basische Gruppe, besonders bevorzugt sind HALS-Amin der Formel XIII (4-Amino-2,2,6,6,-tetramethylpiperidin) oder N,N-Dimethylaminopropylamin (DMAPA) bzw. 3-(Dimethylamino)propylamine (CAS:109-55-7), N-(3-Aminopropyl)imidazole (CAS: 5036-48-6), Dimethylethanolamin (CAS:108-01-0), Dimethylaminoethoxyethanol (CAS:1704-62-7); Trimethylaminoethylethanolamine (CAS: 2212-32-0) oder deren Salzen. Denkbar ist auch eine Umsetzung von seitenkettenständigen, funktionellen Gruppen oder Si-OH-Gruppen mit einem der vorgenannten Amine, vorzugsweise der Formel XIII.

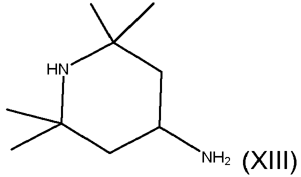

Ebenfalls Gegenstand der Erfindung ist ein Verfahren zur Herstellung eines Siloxan-Polymers, insbesondere der Formel I, vorzugsweise der Formel XI, sowie Siloxan-Polymere und Zusammensetzungen enthaltend diese Siloxan-Polymere erhältlich nach dem Verfahren mit einem zentralen Polysiloxan-Polymerblock B, insbesondere eines Verfahrens zur Herstellung mindestens eines Siloxan-Polymers der allgemeinen Formel (I), wie vorstehend beschrieben, sowie von Zusammensetzungen enthaltend diese Siloxan-Polymere oder Mischungen der Siloxan-Polymere mit einem zentralen Polysiloxan-Polymerblock B, indem a) ein Polysiloxan-Diisocyanat der Formel VII,

OCN-Q2"-D-(O=C)-A-Q2'-B-Q1'-A-(C=O)-D-Q1"-NCO (VII)

mit mindestens einem Hydroxy- oder Amino-funktionellen UV/Vis-Chromophor oder Salz davon umgesetzt wird, wobei die Umsetzung vorzugsweise im molaren Verhältnis von mindestens 1 zu 1 in Bezug auf die Isocyanat-Gruppen des Polysiloxans zu Amino- oder Hydroxy-Gruppen der Chromophore erfolgt,
und ein Siloxan-Polymer der allgemeinen Formel (I)

Q2-B-Q1 (I)

erhalten wird, wobei -Q1 der allgemeinen Formel Ila und -Q2 der Formel IIb entsprechen,

-Q1 =-Q1'-A-(C=O)-D-Q1"-A'-(C=O)-D'-Q1* (IIa)

-Q2 = -Q2'-A-(C=O)-D-Q2"-A'-(C=O)-D'-Q2* (IIb)

- mit A gleich -NH-, -O- oder -S- und D gleich -NH- jeweils unabhängig in Formeln IIa und IIb,
- mit A' gleich -NH- und D' gleich -NH-, -O- oder -S- jeweils unabhängig in Formeln IIa und IIb, wobei jeder Rest Q1 und Q2 der Formel IIa oder IIb jeweils unabhängig mindestens zwei bivalente Gruppen ausgewählt aus Carbamat- und
Harnstoff-Gruppe aufweist, insbesondere weist jeder Rest -Q1 und -Q2 zwei Carbamat-Gruppen oder eine Carbamat und eine Harnstoff-Gruppe oder auch zwei Harnstoff-Gruppen auf, oder
b) ein Polysiloxan der Formel VI

HA-Q2'-B-Q1'-AH (VI)

mit einem UV/Vis-Chromophor-Isocyanat, insbesondere ein UV-Chromophor-Isocyanat ausgewählt aus Cumarin-Isocyanaten, ausgewählt aus den Formeln IXa, IXb, IXc und IXd

Q2*-O(CO)NH-"2Q-NCO (IXa) Q1*-O(CO)NH-"1Q-NCO (IXb) Q2*-O(CO)NH-Q2"-NCO (IXc) Q1*-O(CO)NH-Q1"-NCO (IXd)

umgesetzt wird,
- mit A gleich -NH-, -O- oder -S- und D gleich -NH- jeweils unabhängig in Formeln VII, I, VI, IXa, IXb, IXc und IXd, insbesondere umfassend IXa und IXb,
- mit Q1' und Q2' jeweils unabhängig umfassend einen bivalenten Kohlenwasserstoff-Rest mit 6 bis 200 C-Atomen optional umfassend mindestens ein Heteroatom umfassend O, N oder S, einen bivalenten Rest umfassend Aryl-, Arylalkyl-Gruppen oder einen bivalenten Rest
   umfassend Aryl-, Arylalkyl-Gruppen optional umfassend mindestens ein Heteroatom O, N oder S oder Alkyl-, Aryl- oder Alkyl- und Aryl- Gruppen enthaltende Polyether-Reste, jeweils unabhängig in Formeln VII, I und/oder VI,
- mit Q1" und Q2" jeweils unabhängig umfassend einen bivalenten linearen, verzweigten und/oder cyclischen Alkyl-Rest mit 4 bis 200 C-Atomen, insbesondere einem cyclischen C6 Alkyl-Rest mit Alkylseitenketten, oder einen bivalenten Rest umfassend einen Aryl- und/oder Arylalkyl-Rest mit 6 bis 200 C-Atomen, jeweils unabhängig in Formeln VII, I IVa und/oder IVb,
- mit -O-Q1* oder -O-Q2*für d'-Q1*, D'-Q2* jeweils unabhängig als -Q1* und -Q2* ein UV/Vis-Chromophor als Rest, insbesondere ein UVA2-Chromophor, insbesondere mit mindestens einem Absorptionsmaximum von 320 bis 340 nm, und/oder UVB-Chromophor, insbesondere mit mindestens einem Absorptionsmaximum von 280-320 nm, das vorzugsweise ein Cumarin, Cumarin-Derivat oder ein Salz davon umfasst.

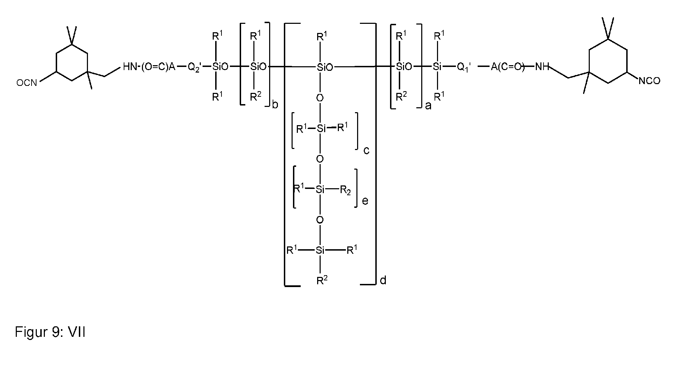

Figur 9 stellt ein erhältliches Diisocyanat nach Formel VII mit D jeweils gleich -NH- dar. Vorzugsweise ist (i) das Hydroxy- oder Amino-funktionelle UV/Vis-Chromophor ein UV-Chromophor umfassend Hydroxy-Cumarin, Amino-Cumarin oder ein Derivat von Cumarin, ii) das UV/Vis-Chromophor-Isocyanat ist ein UV-Chromophor, ausgewählt aus Cumarin-Isocyanaten aus den Formeln IXa, IXb, IXc und IXd, mit -O-Q1* und -O-Q2* jeweils unabhängig als Rest -Q1* und/oder -Q2* Cumarin oder Cumarin-Derivat, und/oder (iii) in Formel IIa, IIb, IXa, IXb, IXc und/oder IXd und I umfassen -O-Q1* und -O-Q2* jeweils unabhängig Reste aus der Umsetzung von Hydroxycumarin oder Hydroxycumarin-Derivat, insbesondere 7-Hydroxycumarin, 4-Hydroxycumarin, 6-Hydroxycumarin oder Hydroxy-Isocumarin mit einem Diisocyanat.

Die Umsetzung in Schritt a) kann in Gegenwart eines Katalysators ablaufen, wie den im Stand der Technik bekannten Katalysatoren zur Polyurethanherstellung und Isocyanattrimerisierung. Beispielhaft genannt sind tertiäre Amine wie Triethylamin, Tetraethylendiamin, oder starke Basen wie DBU, sowie Zinn- und Bismuthverbindungen, wie beispielsweise Dibutylzinnlaurat oder Zinn(II)-octoat.

Nach einer Verfahrensvariante umfassen die Formel IVa, IVb und I als -ONH-Q1* und, -O-Q2* jeweils unabhängig Reste, die aus einem Hydroxy-funktionellen 7-Cumarin, 4-Cumarin, 6-Cumarin abgeleitet sind.

Das erfindungsgemäße Verfahren kann insbesondere die folgenden Schritte umfassen sowie einzelne Schritte umfassen:
(I) H-B-H (x) + Q1'-AH (VIIIa), Q2'-AH (VIIIb) → HA-Q2'-B-Q1'-AH (VI)
(II) HA-Q2'-B-Q1'-AH + Diisocyanat → OCN-Q2"-D-(O=C)-A-Q2'-B-Q1'-A-(C=O)-D-Q1"-NCO (VII)
   mit -AH gleich -NH₂ oder -OH
(III) OCN-Q2"-D-(O=C)-A-Q2'-B-Q1'-A-(C=O)-D-Q1 "-NCO (VII) + Cumarin-Derivat → Q2-B-Q1 (I)
oder alternativ
(la) Diisocyanat (bspw. IPDI) + Hydroxy-Cumarin-Derivat → Q2*-O(CO)NH-"2Q-NCO (IXa) + Q1*-O(CO)NH-"1Q-NCO (IXb) und optional Q2*-O(CO)NH-Q2"-NCO (IXc) und/oder Q1*-O(CO)NH-Q1"-NCO (IXd)
(Ib) H-B-H (x) + Q1'-AH (VIIIa), Q2'-AH (VIIIb) → HA-Q2'-B-Q1'-AH (VI)
(II) HA-Q2'-B-Q1'-AH (VI) + Q2*-O(CO)NH-"2Q2-NCO (IXa)/ Q1*-O(CO)NH-"1Q-NCO (IXb) → Q2-B-Q1 (I)

Zur Herstellung der Siloxan-Polymere kann zunächst
(i) ein Polysiloxan-Gruppen enthaltender linearer und/oder verzweigter Polymerblock B, insbesondere der Formeln IIIa und/oder IIIb oder der Formel XI mit R¹⁷ =H und a2 größer gleich 2, b1 größer gleich 1 oder a1 größer gleich 1, a2 größer gleich 1 und b1 größer gleich 1, mit mindestens zwei terminalen Si-H-Gruppen oder mindestens einer terminalen Si-H-Gruppe und mindestens einer seitenkettenständigen Si-H-Gruppe, bspw. H-B-H (X), wobei -H zwei Si-H Gruppen entspricht, umgesetzt werden
(ii) mit einer olefinischen Verbindung umfassend Alkylen und optional mindestens ein Heteroatom wie N, O, S, insbesondere Alkenylenol, Alkylenamin, Alkylencarbonsäure, Alkylenester, Alkylenamid oder einen olefinischen Polyether umgesetzt wird, wobei die olefinische Verbindung jeweils unabhängig eine Allyl- oder Vinyl-Gruppe aufweist und den Formeln VIIIa und/oder Vlllb entspricht

   Q1' -AH (VIIIa) Q2'-AH (VIIIb)

   mit Q1' und Q2' jeweils unabhängig umfassend ein Alkenylen mit 6 bis 200 C-Atomen optional umfassend mindestens ein Heteroatom O oder N, Aryl- oder Arylalkyl-Gruppen optional umfassend mindestens ein Heteroatom O oder N, olefinischen Polyether mit -AH in Formeln VIIIa und Vlllb unabhängig ausgewählt aus
   sind aus -OH und -NH₂. Die Umsetzung erfolgt vorzugsweise in (iii), in Gegenwart eines Katalysators, wie einem Karstedt-Katalysator, zu einem Polysiloxan der Formel VI,

   HA-Q2'-B-Q1'-AH (VI)

   wobei jeweils unabhängig in Formeln VIIIa, Vlllb und VI mit AH unabhängig ausgewählt aus -OH und -NH₂, und mit -Q2'- und -Q1'- jeweils unabhängig umfassend einen bivalenten Kohlenwasserstoff-Rest mit 6 bis 200 C-Atomen optional umfassend mindestens ein Heteroatom O oder N einen bivalenten Rest umfassend Aryl-, Arylalkyl-Gruppen optional umfassend mindestens ein Heteroatom O oder N oder olefinischen Polyether.

Zur Umsetzung von olefinischen Verbindungen mit der Si-H-Gruppe werden Hydrosilylier-Katalysatoren eingesetzt. Üblich ist die Verwendung eines Karstedt-Katalysators. Generell sind Platinkatalysatoren bevorzugt, bei denen Platin(0) vorliegt.

Mercaptoalkyl substituierte Siloxane, insbesondere der Formel I, VI oder XI kann der Fachmann nach ihm aus dem Stand der Technik bekannten Verfahren über eine Kondensation und/oder Equilibrierung herstellen.

In einem nachfolgenden Verfahrensschritt kann das Polysiloxan der Formel VI

HA-Q2'-B-Q1'-AH (VI)

mit A ausgewählt aus -O, -NH,-S- bzw. AH ausgewählt aus -OH, -NH₂, und -SH mit - Q2'- und -Q1'- wie vorstehend definiert, umgesetzt werden mit einem Diisocyanat zu einem Polysiloxan-Diisocyanat der Formel VII, vorzugsweise ist das Diisocyanat IPDI,

OCN-Q2"-D-(O=C)-A-Q2'-B-Q1'-A-(C=O)-D-Q1 "-NCO (VII)

mit -Q2"- und/oder -Q1"- unabhängig ausgewählt aus einem bivalenten, linearen, verzweigten und/oder cyclischen Alkyl-Rest mit 4 bis 200 C-Atomen, insbesondere ein Isophoron-Rest, oder einem bivalenten Rest umfassend einen Aryl- und/oder Arylalkyl-Rest mit 6 bis 200 C-Atomen, wobei das molare Verhältnis von HA-Gruppen im Polysiloxan zu Isocyanat-Gruppen mindestens 1 zu 1 beträgt, insbesondere ist das Verhältnis 1 : 100 bis 1 : 1, vorzugsweise 1 : 10 bis 1 : 1.

Besonders bevorzugt wird ein Diisocyanat mit einem Hydroxy-funktionellen UV/Vis-Chromophor, insbesondere Hydroxy-funktionellen UV-Chromophor zu einem UV-Chromophor-Isocyanat umgesetzt, bspw. gemäß den Formeln IXa und IXb.

Im nächsten Verfahrensschritt kann das hergestellte Polysiloxan-Diisocyanat der Formel VII oder ein beliebiges über ein anderes Verfahren hergestelltes Polysiloxan-Diisocyanat der Formel VII

OCN-Q2"-D-(O=C)-A-Q2'-B-Q1'-A-(C=O)-D-Q1 "-NCO (VII)

mit B gleich einem linearen und/oder verzweigten Polysiloxan-Polymerblock B, mit -Q2'- und -Q1'- jeweils unabhängig umfassend einen bivalenten Kohlenwasserstoff-Rest mit 6 bis 200 C-Atomen optional umfassend mindestens ein Heteroatom O, N oder S, einem bivalenten Rest umfassend Aryl-, Arylalkyl-Gruppen optional umfassend mindestens ein Heteroatom O, N oder S, oder Alkyl-, Aryl- oder Alkyl- und Aryl-Gruppen enthaltende Polyether-Reste, mit A jeweils unabhängig gleich -NH-, -O- oder -S- und D gleich -NH- jeweils unabhängig in Formel VII, und mit -Q2"- und/oder -Q1 "- unabhängig ausgewählt aus einem bivalenten, linearen, verzweigten und/oder cyclischen Alkyl-Rest mit 4 bis 200 C-Atomen, oder einem bivalenten Rest umfassend einen Aryl- und/oder Arylalkyl-Rest mit 6 bis 200 C-Atomen,
umgesetzt werden mit einem Hydroxy- oder Amino-funktionellen UV/Vis-Chromophor, insbesondere einem Hydroxy-funktionellen UV-Chromophor, vorzugsweise mit mindestens einem Absorptionsmaximum im Bereich von 280 bis 380 nm und optional mit mindestens einem Absorptionsmaximum von 300 bis 380 nm, vorzugsweise einem Hydroxy-funktionellen Cumarin, Cumarin-Derivat oder einem Salz davon.

Bevorzugt sind Hydroxy-funktionelle UVA2-Chromophore, insbesondere mit mindestens einem Absorptionsmaximum im Bereich von 320 bis 340 nm auf und optional einem weiteren Absorptionsmaximum im Bereich von 280 bis 320, und/oder ein Hydroxy-funktionelles UVB-Chromophor, insbesondere mit mindestens einem Absorptionsmaximum im Bereich von 280-320 nm, vorzugsweise einem Absorptionsmaximum im Bereich von 280 bis 300 und optional einem weiteren von 301 bis 320 nm. Besonders bevorzugt sind Hydroxy-funktionelles Cumarin, Cumarin-Derivat oder ein Salz davon.

Im erfindungsgemäßen Verfahren können di-funktionelle Isocyanate ausgewählt aus der Gruppe umfassend beispielsweise; Toluol-2,4-diisocyanat (TDI), Diphenylmethandiisocyanat oder Methylendiphenyldiisocyanat (MDI), Hexamethylen-diisocyanat (HMDI), 2,2,4-Trimethylhexan-1,6-diisocyanat (TMDI), Polymeres Diphenylmethandiisocyanat (PMDI), Isophorondiisocyanat (IPDI), 4,4'-Diisocyanatodicyclohexylmethan (H12MDI) eingesetzt werden, wobei die aliphatischen Produkte bevorzugt sind, und Isophorondiisocyanat (IPDI) besonders bevorzugt ist.

Einige dieser Isocyanate weisen Stereozentren auf. Insbesondere wird auf die Isomere des Isophorons hingewiesen. Ausdrücklich sind alle denkbaren Isomere im Umfang dieser Erfindung eingeschlossen. So kann beispielsweise Isophorondiisocyanat in ein cis- und ein trans-Isomer unterschieden werden. Besonders bevorzugt ist Isophorondiisocyanat aus einem cis/trans Gemisch von 5:1 bis 1:5, bevorzugt 3:1 bis 1:3, weiter bevorzugt 1:1. Ein besonders bevorzugtes, kommerzielles Produkt besteht aus einem cis/trans Gemisch von 3:1. Der Einsatz von kommerziellem Isophorondiisocyanat ist bevorzugt. Isophorondiisocyanat ist unter anderen Bezeichnungen erhältlich, welche als Synonyma im Umfang dieser Erfindung eingeschlossen sind: 3-Isocyanatmethyl-3,5,5-trimethylcyclohexylisocyanat, 5-Isocyanato-1-(isocyanatomethyl)-1,3,3-trimethylcyclohexane, CA RN: 4098-71-9. Es sind diverse Handelsnamen üblich, häufig enthalten sie den Namen des Stammmoleküls Isophoron, es sind jedoch auch andere Handelsnamen geläufig: z.B. Desmodur®I (BAYER), Isocur IPDI 22-200 (ISO-ELEKTRA), VESTANAT® IPDI (EVONIK INDUSTRIES), welche ebenfalls im Umfang der vorliegenden Erfindung eingeschlossen sind. Übliche Spezifikationen für Isophorondiisocyanat sind: Gesamtchlorgehalt <400 mg/kg, hydrolysierbares Chlor <200 mg/kg, Reinheit >99,5 Gew.-%, Brechungsindex n²⁵D 1,483 (DIN 51 423, Teil 2), NCO-Gehalt 37,5 - 37,8 Gew.-% (EN ISO 11 909 / ASTM D 2572), das kommerzielle Produkt wird als farblos bis leicht gelb beschrieben. Die genannten Isocyanate können optional zumindest teilweise Prepolymere umfassen.

Als Isocyanat-Gruppen haltige Verbindungen eignen sich alle bekannten Isocyanate. Bevorzugt im Sinne der erfindungsgemässen Lehre sind z. B. aromatische, aliphatische und cycloaliphatische Polyisocyanate mit einer zahlenmittleren Molmasse von unter 800 g/mol. So sind beispielsweise geeignet Diisocyanate ausgewählt aus der Reihe 2,4-/2,6-Toluoldiisocyanat (TDI), Methyldiphenyldiisocyanat (MDI), Triisocyanatononan (TIN), Naphthyldiisocyanat (NDI), 4,4'-Diisocyanatodicyclohexylmethan, 3-Isocyanatomethyl-3,3,5-trimethylcyclohexylisocyanat (Isophorondiisocyanat = IPDI), Tetramethylendiisocyanat, Hexamethylendiisocyanat (HDI), 2-Methylpentamethylendiisocyanat, 2,2,4-Trimethyl-hexamethylendiisocyanat (THDI), Dodecamethylendiisocyanat, 1,4-Diisocyanato-Cyclohexan, 4,4'-Diisocyanato-3,3'-dimethyldicyclohexylmethan, 4,4'-Diisocyanatodicyclohexylpropan(2,2), 3-Isocyanatomethyl-1-methyl-1-isocyanatocyclohexan (MCI), 1,3-Diisooctylcyanato-4-methyl-cyclohexan, 1,3-Diisocyanato-2-methyl-cyclohexan und [alpha],[alpha], [alpha]',[alpha]'-Tetramethyl-m-oder -p-xylylen-diisocyanat (TMXDI), sowie aus diesen Verbindungen bestehenden Gemischen.

Bevorzugte Ausgangsstoffe für die Herstellung der Urethan-Gruppen und vorzugsweise der Harnstoff-Gruppen haltigen Verbindungen sind Isophorondiisocyanat (IPDI) und/ oder 4,4'-Diisocyanatodicyclohexylmethan.

Bevorzugt werden die folgenden Hydroxy-Cumarin oder Hydroxy-Cumarin in dem Verfahren eingesetzt, und insbesondere wird die Hydroxy-Gruppe mit einer Isocyanat-Gruppe umgesetzt. Bei der Umsetzung mit Diisocyanaten wird ein Cumarin-Isocyanat erhalten.

Gegenstand der Erfindung sind auch Zusammensetzungen erhältlich nach dem erfindungsgemäßen Verfahren, insbesondere umfassend Siloxan-Polymere mit mindestens zwei Carbamat-Gruppen, einer Harnstoff- und einer Carbamat-Gruppe oder zwei Harnstoff-Gruppen je organofunktionellem Rest und umfassend ein UV-Chromophor

Entsprechend einer weiteren Ausführungsform ist Gegenstand der Erfindung eine Zusammensetzung enthaltend a) Siloxan-Polymere der allgemeinen Formel XI sowie Mischungen dieser oder b) Siloxan-Polymere mit einem zentralen Polysiloxan-Polymerblock B ausgewählt aus (i) mindestens ein Siloxan-Polymer der allgemeinen Formel I sowie Mischungen umfassend dieses Polymer,
(ii) mindestens ein Siloxan-Polymer der allgemeinen Formel Ia sowie Mischungen umfassend dieses Polymer oder
(iii) mindestens ein Siloxan-Polymer der allgemeinen Formel Ib sowie Mischungen umfassend dieses Polymer sowie der Formel Ia* und/oder Ib* als auch deren Mischungen oder Mischungen enthaltend diese.

Ein weiterer Gegenstand der Erfindung ist ein Zwischenprodukt zur Herstellung von Siloxan-Polymeren, insbesondere der Formel I, ausgewählt aus Cumarin-Isocyanaten ausgewählt aus den Formeln IXa, IXb, IXc und IXd, insbesondere IXa*, IXb*, und optional IXc* und IXd*, oder deren Salzen oder Mischungen der Cumarin-Derivate.

Q2*-O(CO)NH-"2Q-NCO (IXa) Q1*-O(CO)NH-"1 Q-NCO (IXb) Q2*-O(CO)NH-Q2"-NCO (IXc) Q1*-O(CO)NH-Q1"-NCO (IXd)

Mit -"2Q- und -"1Q- mit der Bedeutung, das die sekundären Isocyanat-Gruppen mit der Hydroxy-Gruppe des Cumarins reagiert hat und die primäre -CH₂-NCO-Gruppe später mit einem Polysiloxan der allgemeinen Formel (VI) HA-Q2'-B-Q1'-AH reagieren kann, insbesondere mit B gleich Formel IIIa oder IIIb sowie alternativ in der Bedeutung der Formel XI mit R¹⁷ jeweils unabhängig gleich -Q1'-AH oder HA-Q2'- ungesetzt werden kann.

Mit Q2*, Q2", Q1* und Q1", wie vorstehend definiert, wobei Q2* und Q1* jeweils unabhängig Cumarin oder ein Cumarin-Derivat sind, insbesondere Cumrarin gebunden an C-7, C-4 oder C-6 des Cumarins oder eines Cumarin-Derivates, vorzugsweise Cumarin-Isocyanate der Formeln IXa* und IXb* oder deren Salzen

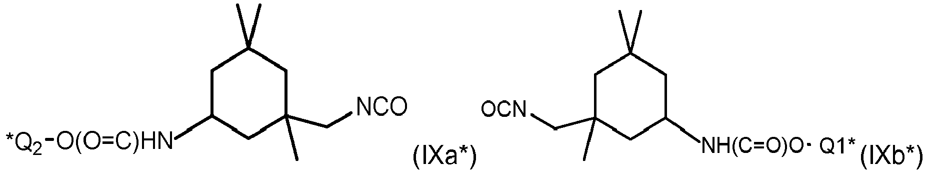

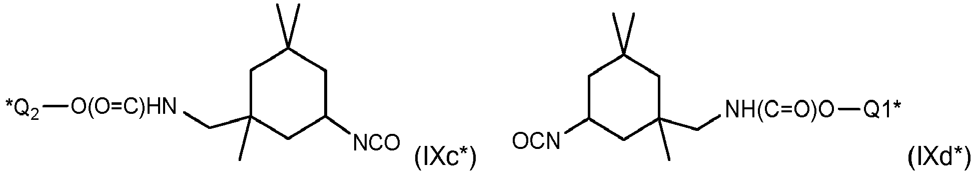

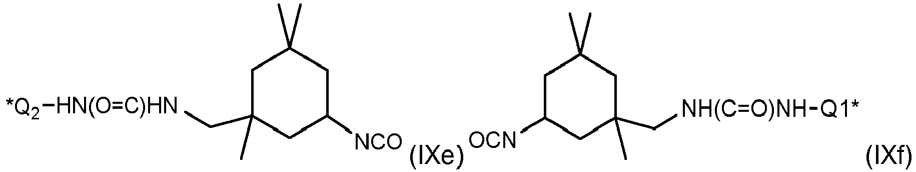

In den Formen IXe und IXf stellen Q2* und Q1* Amino-funktionelle KohlenwasserstoffReste dar, die insbesondere erhältlich sind aus der Umsetzung eines Isophorondiisocyanats (IPDI) mit einem Diamin, insbesondere der Formel XIII HALS-Amin oder N,N-Dimethylaminopropylamin (DMAPA) bzw. 3-(Dimethylamino)-propylamine (CAS:109-55-7), N-(3-Aminopropyl)imidazole (CAS: 5036-48-6), Dimethylethanolamin (CAS:108-01-0), Dimethylaminoethoxyethanol (CAS:1704-62-7); Trimethylaminoethylethanolamin (CAS: 2212-32-0) oder deren Salzen, N,N-Dimethylaminopropylamoin (DMAPA).

Die erfindungsgemäßen Siloxan-Polymere der Erfindung sollen hinsichtlich ihrer UV-Aktivität vorteilhafte Eigenschaften aufweisen. Von besonderem Interesse sind Siloxane, die mit Chromophoren-Gruppen ausgestattet sind um eine Schädigung natürlicher Oberflächen, wie der Haut oder von Haaren vorzubeugen, indem in UV-Bereich nahe des Übergangs von UVB zu UVA bei 315 ein Absorptionsmaximum aufweisen. Ein Additiv mit diesen Eigenschaften wäre in der Lage Haar vor UV-Schädigungen zu schützen, ohne es zu beschweren. Darüber hinaus wäre ein solches Siloxan, insbesondere mit basischen Amino-Gruppen in der Lage sich auf die Haaroberfläche zu legen und durch das UV-Chromophor das Haar vor einer Schädigung durch Sonneneinstrahlung zu schützen. Erfindungsgemäß wird das Polysiloxan mit 7-Hydroxycumarin, 4-Hydroxycumarin oder 6-Hydroxycumarin über ein Diisocyanat an das Siloxan gebunden und dient als Schutz vor Sonneneinstrahlung im Bereich um 315 nm. Ein seitenkettenständiges über eine Harnstoff und Carbamat-Gruppe an Siloxan-Polymere gebundenes Umbelliferon ist in der Lage als UV-Schutz im Bereich von etwa 327 nm in Haarpflegeprodukten eingesetzt zu werden.

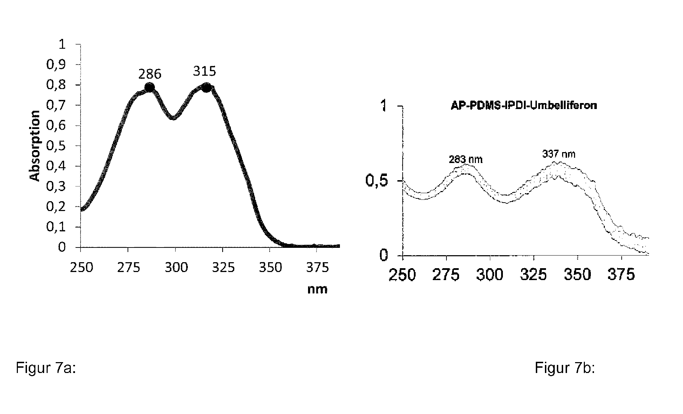

Um nun das Absorptionsmaximum bei einer bestimmten Wellenlänge der mit den chromophoren Substituenten versehenen Verbindung 32 zu bestimmen, wurde diese in Chloroform gelöst und einem UV-Spektrometer vermessen. Die erhaltenen Werte wurden normiert und sind in Abbildung 7a dargestellt. Wie deutlich zu erkennen ist, absorbiert das PDMS-Umbelliferon (32) am stärksten bei 286 bzw. 315 nm. In der Literatur wird u.a. 315 nm als Grenze zwischen dem sogenannten UV-B und UV-A Bereich definiert. Verbindung 32 absorbiert Licht demnach genau in diesem Grenzbereich und entspricht somit den zuvor beschriebenen Anforderungen für die Anwendung als UV-Absorber in Haarpflegeprodukten.

Zusätzlich zu einem UV-Schutz war es ein Anliegen auch Siloxane zu entwickeln, die in der Lage sind das Haar vor freien Radikalen zu schützen. Geeignete Verbindungen sind basische Amine mit sterisch gehinderten Amino-Gruppen wie HALS oder DMAPA-Reste, die an die erfindungsgemäßen Siloxane gebunden werden können.

Daher ist ein Gegenstand der Erfindung eine Formulierung, enthaltend mindestens ein Siloxan-Polymer oder eine Mischung enthaltend mindestens ein Siloxan-Polymer oder über das Zwischenprodukt hergestelltes Siloxan-Polymer und mindestens einen Hilfsstoff. Vorzugsweise ist die Formulierung eine kosmetische Spülung für Haare, pflegendes Haut oder Haarprodukt, Lack, Haarspray, Haarfärbemittel, Farbe, Mundspülung, pharmazeutische Formulierung, Abformmasse (technisch, pharmazeutisch, kosmetisch, dental), Reinigungsmittel, Holzpflegeprodukt, Lackpflege.

Ein weiterer Gegenstand der Erfindung ist die Verwendung der Siloxan-Polymere, der erhältlichen Zusammensetzungen umfassend Siloxan-Polymere nach dem erfindungsgemäßen Verfahren als Additiv in kosmetischen Formulierungen, als Additiv in pharmazeutischen Formulierungen, in Lacken, Pasten, als Schaumstabilisator oder Schaumadditiv für Polyurethanschäume, insbesondere Polyurethanhartschäume und Polyurethanweichschäume, als Griffverbessere oder Imprägniermittel bei der Herstellung von Fasern, Textilien, in kosmetischen Formulierungen zur Behandlung, Nachbehandlung und Schutz von keratinischen Fasern, insbesondere in Haarkonditionierungsformulierungen. sowie Haut und Hautanhangsgebilden, als Additiv in Waschmittel-, Weichspülerformulierungen, in kosmetischen Formulierungen umfassend Cremes, Spülungen, Haarwachsmittel, Waschmittel, Festiger, Pflegespülungen, pflegende Pasten, Sprays, Haarsprays, zur Verbesserung der Kämmbarkeit von keratinischen oder textilen Fasern natürlichen oder synthetischen Ursprungs.

Ein weiterer Gegenstand der vorliegenden Erfindung ist die Verwendung der erfindungsgemäßen Siloxane und/oder der Siloxane erhältlich nach dem erfindungsgemäßen Verfahren zur Herstellung von Formulierungen, insbesondere von Pflege- und Reinigungsformulierungen für die Anwendung im häuslichen und industriellen Umfeld. Bevorzugte Pflege- und Reinigungsformulierungen für die Anwendung im häuslichen und industriellen Umfeld sind in diesem Zusammenhang Textilpflegemittel, wie beispielsweise Weichspüler, und Pflegemittel für harte Oberflächen.

Die generelle Synthese eines Isocyanat-terminierten PDMS erfolgt durch Umsetzung Hydroxy-terminierter PDMS verschiedener Kettenlängen, wie (n= 30 bzw. 80) (Die konkreten Verbindungen sind in den Herstellbeispielen näher erläutert; **14** bzw. **15**) mit Isophorondiisiocyanat (**16**), welches sich auf Grund seiner unterschiedlich reaktiven Isocyanat-Gruppen für eine Funktionalisierung der PDMS besonders empfiehlt. So kann ausgeschlossen werden, dass die Diisocyanat-Komponente zweifach mit den Hydroxylgruppen der PDMS reagiert und keine freie Isocyanat-Gruppe für eine weitere Reaktion mit einem Substituenten zur Verfügung steht. Des Weiteren kann dadurch eine Vergelung des Reaktionsgemisches bedingt durch die Bildung hoher Molekülmassen verhindert werden. Das Isophorondiisocyanat (**16**) wird hierfür zunächst in einer sekurierten Apparatur vorgelegt und unter Zugabe katalytischer Mengen Triethylamin mit dem zugetropften α,ω,-Bis(hydroxyhexyl)-polydimethylsiloxan (PDMS-30 bzw. PDMS-80) (**14** bzw. **15**) in Substanz umgesetzt.

Sofern im Umfang dieser Erfindung auf Naturstoffe Bezug genommen wird, z.B. Aminosäure, sind grundsätzlich alle Isomeren damit gemeint, bevorzugt sind die jeweils in der Natur vorkommenden Isomere, im hier genannten Falle also die alpha-Aminosäuren. Zur Definition von Naturstoffen wird auf den Umfang des "Dictionary of Natural Products", Chapman and Hall/CRC Press, Taylor and Francis Group, z.B. in der online Ausführung von 2011: http://dnp.chemnetbase.com/ verwiesen.

Wo immer Moleküle beziehungsweise Molekülfragmente ein oder mehrere Stereozentren aufweisen oder aufgrund von Symmetrien in Isomere unterschieden werden können oder aufgrund anderer Effekte z.B. eingeschränkter Rotation in Isomere unterschieden werden können, sind alle möglichen Isomere von der vorliegenden Erfindung mit eingeschlossen. Isomere sind dem Fachmann bekannt, in besonderer Weise wird auf die Definitionen von Prof. Kazmaier der Universität des Saarlandes verwiesen, z.B. http://www.uni-saarland.de/fak8/kazmaier/PDF_files/vorlesungen/ Stereochemie%2Strassb%20Vorlage.pdf. Insbesondere sind alle Möglichkeiten, die sich aus den stereochemischen Definitionen der Taktizität ergeben eingeschlossen, z.B. isotaktisch, syndiotaktisch, heterotaktisch, hemiisotaktisch, ataktisch. Bevorzugt im Sinne der Erfindung sind Polyether und Polyetherfragmente mit zumindest teilweiser ataktischer Substituentenfolge.

Die folgenden Beispiele erläutern die erfindungsgemäßen Siloxan-Polymere sowie das erfindungsgemäße Verfahren näher, ohne die Erfindung auf diese Beispiele zu beschränken.

### Beispiele:

### Analytik:

UV-VIS-Spektroskopie: Für die UV-VIS spektroskopischen Messungen wurde ein zweistrahliges Spektrometer der Analytik Jena AG, Modell Specord© 210 Plus, verwendet. Zur Vermeidung von Partikelverunreiniungen der Proben und wurden diese mittels eines Spritzenfilters der Porengröße 0,45 µm filtriert und in eine Quarzglasküvette der Schichtdicke 1 cm überführt. Die Messungen wurden in einem Bereich von 250 bis max. 800 nm und bei einer Temperatur von 25 °C durchgeführt.

MALDI-TOF-MS: Ultraflex time of flight-Massenspektrometer, Bruker. 337 nm Stickstofflaser, Linearmodus oder Reflektormodus. Eingewogene Proben wurden in geeignetem Lösungsmittel gelöst. Als Matrix diente Dithranol (DIT) oder 2,5-Dihydroxybenzoesäure (DHB).

FT-IR-Spektren: FT-IR-5SXB, Nicolet verwendet. Kalibrierung: mittels HeNe-Lasers. ATR-Messungen: specac golden-gate Diamant ATR-Einheit.

NMR-Spektroskopie: 300 MHz-NMR Spektrometer, Bruker, Modell Avance III - 300, Magnetfeldstärke 7.05 Tesla. Absorptionsfrequenz: 1 H-NMR bei 300 MHz, 13C{1H}-NMR bei 75 MHz. 200 bzw. 500 MHz-NMR-Spektren: FT-NMR-Spektrometer, Bruker DRX200 bzw. DRX500.

### Beispiel 1: Synthese Isocyanat-terminierter Polydimethylsiloxane

Die Synthese eines Isocyanat-terminierten PDMS erfolgte durch Umsetzung Hydroxy-terminierter PDMS verschiedener Kettenlängen (n= 30 bzw. 80) (**14** bzw. **15**) mit Isophorondiisiocyanat (**16**). Der Vorteil des IPDIs ist, dass die Diisocyanat-Komponente nicht zweifach mit den Hydroxylgruppen der PDMS reagiert. Des Weiteren konnte eine Vergelung des Reaktionsgemisches bedingt durch die Bildung hoher Molekülmassen verhindert werden. Isophorondiisocyanat (**16**) wird in einer sekurierten Apparatur vorgelegt und katalytische Mengen Triethylamin mit dem zugetropften α,ω-Bis(hydroxyhexyl)-polydimethylsiloxan zugegeben (PDMS-30 bzw. PDMS-80) (**14** bzw. **15**) in Substanz umgesetzt. Die Reaktion der α,ω-Bis(hydroxyhexyl)-polydimethylsiloxane **14** (n=30) und **15** (n=80) mit Isophorondiisocyanat (**16**) zu den α,ω-Bis[hexyl(3-(isocyanatomethyl)-3,5,5-trimethylcyclohexyl)carbamyl]-polydimethylsiloxanen **17** und **18** (PDMS-30-IPDI, n= 30 bzw. PDMS-80-IPDI, n=80) kann vorzugsweise in Gegenwart von Dichlormethan bei 60 °C erfolgen. Eine übliche Reaktionsdauer beträgt 2 Stunden.

Analytik Isocyanat-terminierter PDMS: MS, 1H-NMR-, IR-Spektroskopie, Molmassen (MALDI-TOF) bestätigen Verbindungen **17** und **18**; IR: charakteristische C-H Valenz- und Deformationsschwingungen des Polydimethylsiloxans bei 2961, 1412 und 1257 cm-1, 2256 cm-1 freie Isocyanat-Gruppen, 1709 cm-1 C=O Valenzschwingung Urethaneinheit. Die Umsetzung von PDMS-30-IPDI, n= 30 (**17**) mit Umbelliferon (**31**) zu einem Umbelliferon-terminierten Polydimethylsiloxan (PDMS-Umb) (**32**) kann vorzugsweise in einem Gemisch aus Dichlormethan/DMF bei Raumtemperatur erfolgen. Eine übliche Reaktionsdauer beträgt etwa 24 Stunden.

Das Produkt **32** wurde anschließend mittels 1H-NMR und IR-Spektroskopie sowie MALDI-TOF analysiert. Es wurde ein MALDI-TOF Spektrum des PDMS-Umb (**32**) bestimmt. Die Massenzahlen höhere Intensität entsprechen dabei dem mit Umbelliferon disubstituierten Polydimethylsiloxan-Derivat unterschiedlicher Kettenlängen, welches mit Natrium ionisiert vorliegt. Der Abstand zwischen diesen Massenzahlen kann wiederum genau einer Siloxaneinheit zugeordnet werden. Somit deutet das Ergebnis der massenspektroskopischen Untersuchung auf die gelungene Synthese der Struktur **32** hin. Dies wird anhand der IR-Daten noch einmal bestätigt. Es wird keine Valenzschwingung einer freien Isocyanatgruppe des Eduktes **17** mehr detektiert, was auf eine vollständige Umsetzung der Edukte **17** und **31** zu dem gewünschten Produkt **32** schließen lässt. Des Weiteren beweist die 1H-NMR spektroskopische Analyse die Entstehung des Umbelliferon-terminierten PDMS (**32**).

Um nun das Absorptionsmaximum bei einer bestimmten Wellenlänge der mit den chromophoren Substituenten versehenen Verbindung **32** zu bestimmen, wurde diese in Chloroform gelöst und einem UV-Spektrometer vermessen. Die erhaltenen Werte wurden normiert und sind in Abbildung 7a dargestellt. Wie deutlich zu erkennen ist, absorbiert das PDMS-Umb (**32**) am stärksten bei 286 bzw. 315 nm. In der Literatur wird u.a. 315 nm als Grenze zwischen dem sogenannten UV-B und UV-A Bereich definiert. Verbindung **32** absorbiert Licht demnach genau in diesem Grenzbereich und entspricht somit den zuvor beschriebenen Anforderungen für die Anwendung als UV-Absorber in Haarpflegeprodukten.

### Beispiel 2: Synthese eines Umbelliferon-terminierten Polydimethylsiloxans (PDMS- Umbelliferon) (32)

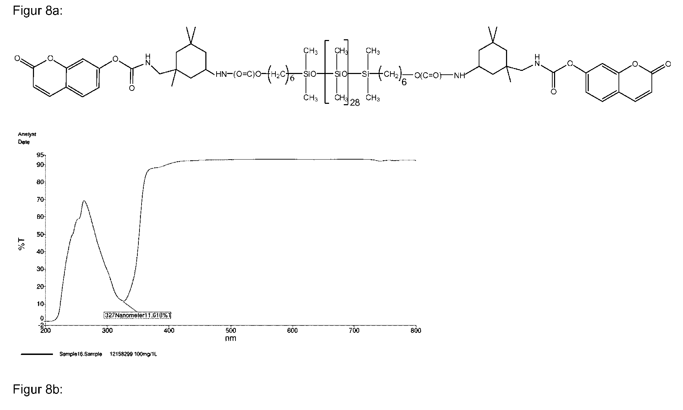

Strukturformel des Umbelliferon-terminierten PDMS: Figur 8a. 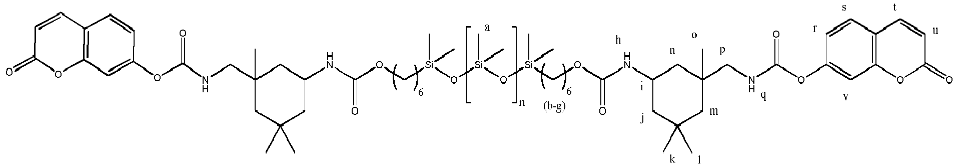 7-Hydroxycumarin (0.39 g, 2.4 mmol) wird in einem sekurierten Rundkolben samt Rückflusskühler und Tropftrichter in einer Mischung aus 20 mL Dichlormethan und 20 mL Dimethylformamid gelöst. Es wird Triethylamin (0.5 mL) hinzugegeben. α,ω- Bis[hexyl(3-(isocyanatomethyl)-3,5,5-trimethylcyclohexyl)carbamyl]- poly(dimethylsiloxan) (n=30) (3.4 g, 1.2 mmol) wird ebenfalls in einer Mischung aus 20 mL Dichlormethan und 20 mL Dimethylformamid gelöst und per Tropftrichter unter Rühren innerhalb von 1 h zugetropft. Nach 24 h Rühren bei 40 °C wird mit je 2x 20 mL dest. Wasser und 1x 20 mL ges. wässr. Natriumhydrogencarbonat-Lösung gewaschen. Die organischen Phasen werden vereinigt, über Natriumsulfat getrocknet und das Lösungsmittel am Rotationsverdampfer entfernt. Das Rohprodukt wird in etwas Ethanol gelöst, in dest. Wasser wieder ausgefällt und zentrifugiert. Abschließend wird das Produkt im Hochvakuum getrocknet.
1 H-NMR (600 MHz, CDCI3, 24 °C): δ [ppm]= 7.59 (2H, m, H-t), 7.39 (2H, m, H-s), 7.07 (4H, m, H-r/H-v), 6.32-6.15 (2H, m, H-u), 3.98 (4H, m, H-g), 3.76 (2H, m, H-i), 2.86 (4H, m, H-p), 1.97-1.44 (8H, m, H-f/H-n), 1.26 (12H, m, H-c/H-d/H-e), 1.15-0.66 (26H, m, H- j/H-k/H-1/H-m/H-o), 0.46 (4H, m, H-b), 0.00 (144H, m, H-a)
FT-IR (Diamant): ü [cm-1]= 2960 (u R-CH3, Si-CH3, m-w), 1666 (u C=C, RCO-O-CH=CH-R, s), 1530 (u C=O, Urethan, m-w), 1437 (δ C-H, Si-CH3, w), 1257 (δ C-H, Siloxan, s-m), 1089 (u Si-O-Si, Siloxan, s-m)
MALDI-TOF-MS m/z: 1505 [M+Na]+ (für n=7), disubstituiert
UV-Spektrum (Chloroform): Figur 7a
UV-Spektrum (Ethanol): Figur 8b
100 mg der Verbindung wurden in 1 I Ethanol gelöst und in einem UV-Spektrometer gemessen. Das Transmissionsminimum (=Absorptionsmaximum) der Verbindung aus Beispiel 2 liegt bei 327 nm.
Ausbeute: 94%

### Beispiel 3: Synthese eines Umbelliferon-funktionalisierten Aminopropyl-Polydimethylsiloxan-Copolymers (AP-PDMS-IPDI- Umbelliferon) (36)

U-IPDI (24.0 mg, 0.06 mmol) wird in einer zuvor sekurierten Apparatur bei RT in 10 mL Dichlormethan vorgelegt. Über einen Tropftrichter wird anschließend das Aminopropyl-Polydimethylsiloxan-Copolymer (10.0 g, 2.0 mmol), welches zuvor ebenfalls in 40 mL Dichlormethan gelöst wurde, innerhalb von 1 h tropfenweise unter Argongegenstrom hinzugegeben. Nach 24 h Rühren bei RT wird das Lösungsmittel am Rotationsverdampfer entfernt und das Produkt abschließend im Hochvakuum getrocknet. 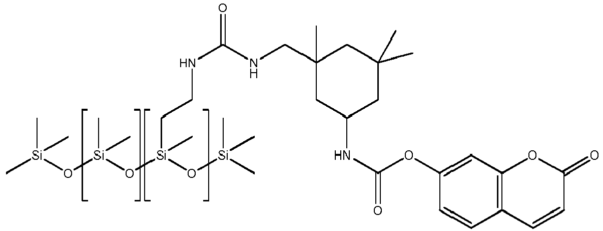 FT-IR (Diamant): ü [cm-1]= 2962 (u R-CH3, Si-CH3, m-w), 2901 (u C-H, -CH2-, m-w), 1590 (u C=C, Aromat, v) (u C=O, N-CO-N, v), 1444 (δ C-H, Si-CH3, w), 1412 (δ C-H, Si-CH3, m-w), 1257 (δ C-H, Siloxan, s-m), 1009 (u Si-O-Si, Siloxan, s-m) UV-Spektrum: Figur 7b

### Beispiel 4: Synthese kurzkettiges α,ω-Bis[hexyl(3-(isocyanatomethyl)-3,5,5-trimethylcyclohexyl)carbamyl]-polydimethylsiloxan (PDMS-30-IPDI) (17), n = 30

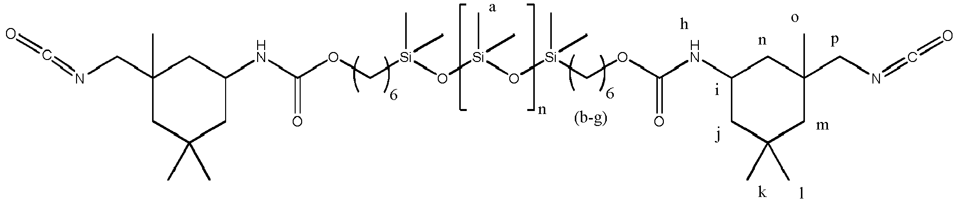

Isophorondiisocyanat (4,72 mL, 22,5 mmol) wird in einem sekurierten Rundkolben mit Rückflusskühler und Tropftrichter vorgelegt. Unter Schutzgasatmosphäre und Rühren wird auf 60 °C erhitzt. 0,1 Gew.-% Triethylamin (32 mg) werden hinzugefügt. Innerhalb von 2 h wird α,ω-Bis(hydroxyhexyl)-poly(dimethylsiloxan) (n=30) (26.4 g, 11.0 mmol) langsam zugetropft. Es wird solange gerührt, bis keine Trübung mehr auftritt. Das Produkt wird in quantitativer Ausbeute erhalten.

1 H-NMR (300 MHz, CDCl3, 24 °C): δ [ppm]= 3.98 (4H, m, H-g), 3.57 (2H, m, H-i), 2.98 (4H, m, H-p), 1.88-1.38 (8H, m, H-f/H-n), 1.26 (12H, m, H-c/H-d/H-e), 1.20-0.72 (26H, m, H-j/H-k/H-l/H-m/H-o), 0.46 (4H, m, H-b), 0.00 (168H, m, H-a); FT-IR (Diamant): ũ [cm-1]= 2961 (u R-CH3, Si-CH3, m-w), 2256 (u -NCO, Isocyanat, s), 1709 (u C=O, Urethan, s), 1412 (δ C-H, Si-CH3, w), 1257 (δ C-H, Siloxan, s-m), 1011 (u Si-O-Si, Siloxan, s-m); MALDI-TOF-MS m/z: 1542 [M+Na]+ (für n=10), disubstituiert

### Beispiel 5: Synthese langkettiges α,ω,-Bis[hexyl(3-(isocyanatomethyl)-3,5,5-trimethylcyclohexyl)carbamyl]-polydimethylsiloxan (PDMS-80-IPDI) (18), Struktur analog Beispiel 4 mit n = 80

Isophorondiisocyanat (4,72 mL, 22,5 mmol) wird in einem sekurierten Rundkolben samt Rückflusskühler und Tropftrichter vorgelegt. Unter Schutzgasatmosphäre und Rühren wird auf 60°C erhitzt. 0,1 Gew.-% Triethylamin (32 mg) werden hinzugegeben. α,ω-Bis(hydroxyhexyl)-poly(dimethylsiloxan) (n=80) (67.1 g, 11.0 mmol) wird innerhalb von 2 h langsam zugetropft. Es wird solange gerührt, bis keine Trübung mehr auftritt. Das Produkt wird in quantitativer Ausbeute erhalten.

1 H-NMR (300 MHz, CDCl3, 24 °C): δ [ppm]= 4.01 (4H, m, H-g), 3.77 (2H, m, H-i), 3.01 (4H, m, H-p), 1.89-1.42 (8H, m, H-f/H-n), 1.33 (12H, m, H-c/H-d/H-e), 1.24-0.76 (26H, m, H-j/H-k/H-l/H-m/H-o), 0.51 (4H, m, H-b), 0.00 (396H, m, H-a); FT-IR (Diamant): ũ [cm-1]= 2960 (u R-CH3, Si-CH3, m-w), 2256 (u -NCO, Isocyanat, s), 1711 (u C=O, Urethan, s), 1411 (δ C-H, Si-CH3, w), 1257 (δ C-H, Siloxan, s-m), 1010 (u Si-O-Si, Siloxan, s-m); MALDI-TOF-MS m/z: 2283 [M+Na]+ (für n=20), disubstituiert

### Beispiel 6: Synthese von α,ω-Bis[hexyl(6-isocyanatohexyl)carbamyl]poly-(dimethylsiloxan)

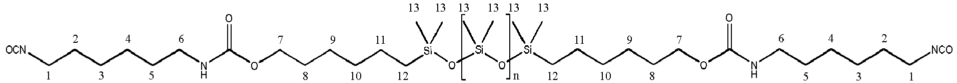

2,00 g (11.89 mmol) 1,6-Hexamethylendiisocyanat, gelöst in 20 mL Ethylacetat, werden in einer ausgeheizten, mit Argon gespülten und sekurierten Apparatur mit Rückflusskühler und Tropftrichter vorgelegt. Man erhitzt unter Rühren auf 75 °C und gibt anschließend 0,1 Gew.-% (16 mg) Triethylamin hinzu. Per Tropftrichter werden anschließend 14 g (5.8 mmol) α,ω-Bis(hydroxyhexyl)-poly(dimethylsiloxan), gelöst in 30 mL Ethylacetat, zur Diisocyanat-Komponente langsam zugegeben (Tropfzeit 2 Std.). Dabei wird stets gerührt. Anschließend wird für weitere 16 Std. bei 75 °C gerührt. Ausbeute: 15,90 g

¹H-NMR: (300 MHz, CDCl₃) δ = 4.01 (m, 4H, 7), 3.27 (q, 4H, 1), 3.13 (m, 4H, 6), 1.68-1.23 (m, 32H, 2, 3, 4, 5, 8, 9, 10, 11), 0.50 (m, 4H, 12), 0.04 (m, 180H, 13) ppm. FT-IR (Diamant): ṽ = 2964 v(C-H), 2267 v(NCO), 1708 v(C=O)_{Urethan}, 1523 δ(N-H), 1410 δₐₛ(C-H), 1254 δ_{sym}(C-H), 1011 v(Si-O-C), 853 + 789 v(SI-C) cm⁻¹.

Die Verbindungen der Beispiele 4, 5 und 6 können anschließend mit Hydroxy- oder Amino-funktionellen Chromophoren zu den erfindungsgemäßen Siloxan-Polymeren umgesetzt werden.

### Beispiel 7: Synthese von Isocyanat-funktionalisierten Cumarin-Derivaten (C-IPDI, IXa*, IXb*)

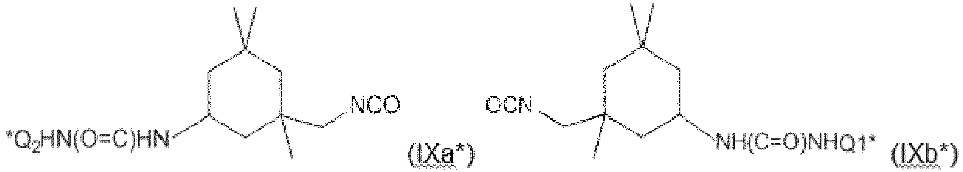

sowie ggf. der allgemeinen Formeln IXc* und/oder IXd* oder Mischungen davon.

### Beispiel 8: Synthese eines Isocyanat-funktionalisierten Cumarin-Derivat (C-IPDI), Cumarin-IPDI, insbesondere Synthese eines Isocyanat-funktionalisierten Umbelliferon-Derivats (U-IPDI) (34)

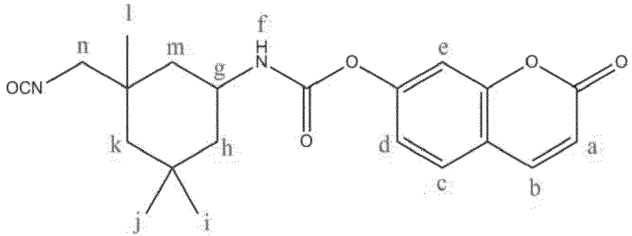

Isophorondiisocyanat (4.72 mL, 22.5 mmol) wird in einer zuvor sekurierten Apparatur bei RT vorgelegt. Es werden 1-2 Tropfen Triethylamin unter Argongegenstrom hinzugefügt. Über einen Tropftrichter wird anschließend 7-Hydroxycumarin (3.65 g, 22.5 mmol), welches zuvor in einer Mischung aus 10 mL Dichlormethan und 10 mL Dimethylformamid gelöst wurde, innerhalb von 2 h tropfenweise zu der Reaktionsmischung gegeben. Nach 24 h Rühren bei RT werden die Lösungsmittel am Rotationsverdampfer entfernt und das Produkt abschließend im Hochvakuum getrocknet.

1 H-NMR (300 MHz, CDCl3, 24 °C): δ [ppm]= 7.65 (1 H, d, H-b), 7.41 (1 H, m, H-c), 7.08 (2H, m, H-d/H-e), 6.32 (1 H, d, H-a), 3.90-3.27 (3H, m, H-g/H-n), 1.78 (2H, m, H-m), 1.28-0.78 (13H, m, H-i/H-j/H-k/H-I); FT-IR (Diamant): ü [cm-1]= 3291 (u N-H, Urethan, w), 2927 (u C-H, R-CH2-R, m-w), 2255 (u -NCO, Isocyanat, s), 1735 (u C=O, Urethan, s), 1660 (u C=O, R-CO-O-R, s), 1619 (u C=C, Aromat, v), 1537 (u C=O, Urethan, m-w), 1385 (δ C-H, R-CH3, s-m) (u -NCO, Isocyanat, m-w), 1222 (u C-O, R-O-Ar, s), 1152 (u C-O, R-O-Ar, m);
ESI-lon-Trap-MS m/z (%): 385 [M]+

Entsprechend der Darstellung nach Beispiel 8 kann auch ein Diisocyanat, insbesondere IPDI, mit 4-Amino-2,2,6,6-tetramethylpiperidin zu einem Piperidin-Isocyanat der allgemeinen Formel IX als Zwischenprodukt zur Herstellung von seitenketten- oder terminal substituierten Polysiloxanen hergestellt werden.

### Beispiel 9: Synthese eines Tetramethylpiperidin-terminierten Polydimethylsiloxans (PDMS-4-ATMP) (26)

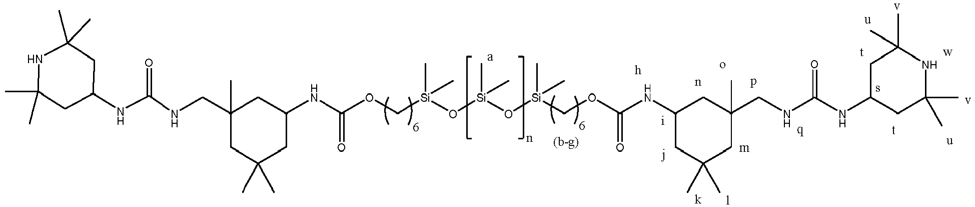

4-Amino-2,2,6,6-tetramethylpiperidin (0.60 mL, 3.5 mmol) wird in einem sekurierten Rundkolben samt Rückflusskühler und Tropftrichter in 20 mL Dichlormethan gelöst. Es wird Triethylamin (0.625 mL, 4.5 mmol) hinzugegeben. α,ω-Bis[hexyl(3-(isocyanatomethyl)-3,5,5-trimethylcyclohexyl)carbamyl]-poly(dimethylsiloxan) (n=30) (4.26 g, 1.5 mmol) wird ebenfalls in 20 mL Dichlormethan gelöst und per Tropftrichter unter Rühren innerhalb von 1 h zugetropft. Nach 24 h Rühren bei RT wird mit je 2x 20 mL dest. Wasser und 1x 20 mL ges. wässr. Natriumhydrogencarbonat-Lösung gewaschen. Die organischen Phasen werden vereinigt, über Natriumsulfat getrocknet und das Lösungsmittel am Rotationsverdampfer entfernt. Das Produkt wird abschließend im Hochvakuum getrocknet. Ausbeute: 77%

1 H-NMR (300 MHz, CDCl3, 24 °C): δ [ppm]= 3.96 (6H, m, H-g/H-s), 3.88-3.50 (2H, m, H-i), 3.24-2.74 (4H, m, H-p), 1.85 (2H, m, H-t), 1.76-1.43 (12H, m, H-f/H-n), 1.30-0.71 (66H, m, H-c/H-d/H-e H-j/H-k/H-I/H-m/H-o/H-t/H-u/H-v), 0.46 (4H, m, H-b), 0.00 (168H, m, H-a); FT-IR (Diamant): ũ [cm-1]= 2961 (u R-CH3, Si-CH3, m-w), 2923 (u C-H, - CH2-, m-w), 1701 (u C=O, Urethan, m-w), 1630 (u C=O, N-CO-N, v), 1559 (u C=O, N-CO-N, v) (δ N-H, Amin, w), 1410 (δ C-H, Si-CH3, w), 1257 (δ C-H, Siloxan, s-m), 1012 (u Si-O-Si, Siloxan, s-m); MALDI-TOF-MS m/z: 1781 [M+Na]+ (für n=9), disubstituiert

### Beispiel 10: Anwendungstest Sensorik:

Austestung der Konditionierung von Haar mittels Sensoriktests in einer Haarspülung:

Für die anwendungstechnische Beurteilung der Konditionierung von Haar wurden die erfindungsgemäßen Verbindungen Synthesebeispiele 2 (PDMS-Umbelliferon) und 9 (PDMS-4-ATMP) sowie das kommerziell erhältliche Produkt ABIL® Quat 3272 (INCI: Quaternium-80, Hersteller Evonik Industries) in einer einfachen kosmetischen Haarspülungs-Formulierung eingesetzt.

Die anwendungstechnischen Eigenschaften der erfindungsgemäßen Verbindungen beim Einsatz in Haarspülungen wurden in den folgenden Rezepturen überprüft (Angaben in Gew.-% bezogen auf die Gesamtformulierung, mit Wasser wurde zu 100% aufgefüllt):

| **Formulierungsbeispiele** | **0a** | **1a** | **2a** | **V3a** |
|---|---|---|---|---|
| TEGINACID^{®} C, Evonik Industries (INCI: Ceteareth-25) | 0,5% | 0,5% | 0,5% | 0,5% |
| TEGO^{®} Alkanol 1618, Evonik Industries (INCI: Cetearyl Alcohol) | 5,0% | 5,0% | 5,0% | 5,0% |
| VARISOFT^{®} 300, 30%-ig, Evonik Industries (INCI: Cetrimonium Chloride (=CTAC)) | 2,0% | 2,0% | 2,0% | 2,0% |
| Neolone PE, The Dow Chemical Company (INCI: Phenoxyethanol; Methylisothiazolinone) | 0,45% | 0,45% | 0,45% | 0,45% |
| Wasser, demineralisiert | ad. 100,0% | | | |
| Zitronensäure | ad. pH 4,5 ± 0,3 | | | |
| Synthesebeispiel 2 (PDMS-Umbelliferon) | | 0,30% | | |
| Synthesebeispiel 9 (PDMS-4-ATMP)] | | | 0,30% | |
| ABIL^{®} Quat 3272, 50%ig in Propylenglycol (nicht erfindungsgemäß) | | | | 0,60% |

Für die anwendungstechnische Beurteilung wurden Haartressen (Fa. Kerling, Deutschland) durch eine Bleichbehandlung standardisiert vorgeschädigt. Dazu wurden friseurübliche Produkte verwendet. Der Testablauf, die verwendeten Basismaterialien sowie die Details der Beurteilungskriterien wurden in DE 103 27 871 beschrieben.

Die Vorbehandlung der Haare erfolgte durch ein Shampoo, welches keine Konditioniermittel enthält.

Standardisierte Behandlung von vorgeschädigten Haartressen mit konditionierenden Formulierungen:

Die, wie oben beschrieben, vorgeschädigten Haartressen wurden wie folgt mit den oben beschriebenen konditionierenden Spülungen behandelt:

Die Haartressen wurden unter fließendem, warmem Wasser (38 °C, 10 °dH) benetzt. Das überschüssige Wasser wurde leicht von Hand ausgedrückt, dann wird das Shampoo aufgebracht und sanft im Haar eingearbeitet (1 ml/Haartresse (2 g)). Nach einer Verweilzeit von 1 min wurde das Haar für 1 min gespült. Direkt im Anschluss wurde die Spülung aufgebracht und sanft im Haar eingearbeitet (1 ml/Haartresse (2 g)). Nach einer Verweilzeit von 1 min wurde das Haar für 1 min gespült.

### Beurteilungskriterien:

Die sensorischen Bewertungen erfolgten nach Noten, die auf einer Skala von 1 bis 5 vergeben wurden, wobei 1 die schlechteste und 5 die beste Bewertung ist. Die einzelnen Testkriterien erhielten jeweils eine eigene Bewertung.
Die Testkriterien sind: Nasskämmbarkeit, Nassgriff, Trockenkämmbarkeit, Trockengriff, Aussehen/Glanz.

In der folgenden Tabelle wurden die Ergebnisse der sensorischen Beurteilung der wie oben beschrieben mit den erfindungsgemäßen Formulierungen 1 a und 2a, der Vergleichsformulierung V3a und der Kontrollformulierung 0a (Placebo ohne konditionierende Silicon-Testsubstanz) behandelten Haarsträhnchen verglichen.

| | Nasskämmbarkeit | Nassgriff | Trockenkämmbarkeit | Trockengriff | Glanz |
|---|---|---|---|---|---|
| Kontrollformulierung 0a | 3,9 | 3,8 | 3,9 | 4,0 | 3,0 |
| Erfindungsgemäße Formulierung 1a | 4,5 | 4,4 | 4,5 | 4,4 | 4,0 |
| Erfindungsgemäße Formulierung 2a | 4,6 | 4,4 | 4,6 | 4,7 | 4,0 |
| Vergleichsformulierung (nicht erfindungsgemäß) V3a | 4,6 | 4,3 | 4,4 | 4,2 | 3,5 |

Die erfindungsgemäßen Formulierungen 1a und 2a mit den erfindungsgemäßen Verbindungen Beispiel 2 und 9 zeigten in der sensorischen Beurteilung gute kosmetische Bewertungen. Die Kontrollformulierung 0a (mit CTAC) wurde durch die Zugabe von lediglich 0,3% aktiv Siliconprodukt signifikant verbessert. Dabei wurden die bereits sehr guten Eigenschaften der Vergleichsformulierung V3a vor allem bzgl. Trockenkämmbarkeit und Trockengriff durch die erfindungsgemäßen Formulierungen 1a und 2a noch weiter gesteigert. Eine signifikant bessere Bewertung wurde auch beim Glanz durch die Verwendung der erfindungsgemäßen Formulierungen 1a und 2a erreicht. Die besseren Resultate im vergleich zur Vergleichsformulierung V3a ist insofern überraschend, da das Vergleichsprodukt ABIL® Quat 3272 die gleiche Siliconkettenlänge wie die erfindungsgemäßen Beispiele 2 und 9 aufweist.

### Beispiel 11: Weitere Formulierungsbeispiele:

Die in den nachfolgenden Tabellen angegebenen Formulierungsbeispiele zeigen exemplarische Vertreter einer Vielzahl von möglichen erfindungsgemäßen Zusammensetzungen.

Falls die Herstellung der Formulierung zuvor die getrennte Zubereitung bzw. Mischung von Formulierungsbestandteilen erfordert, wird dieses als mehrphasige Zubereitung bezeichnet.

Falls eine zweiphasige Herstellung erforderlich ist, werden die beiden Phasen mit A und B in den angegebenen Tabellen gekennzeichnet. Bei dreiphasigen Prozessen werden die drei Phasen mit A, B und C benannt. Wenn nicht anders angegeben handelt es sich bei den Angaben in den folgenden Tabellen um Angaben in Gew.-% bezogen auf die Gesamtformulierung, mit Wasser wurde zu 100% aufgefüllt, q.s. bedeutet, dass so viel wie notwendig zugesetzt wird und dass diese Menge von der Menge Wasser subtrahiert wird:

**Formulierungsbeispiel 1) Shampoo**

| | |
|---|---|
| TEXAPON^{®} NSO, BASF, 28%-ig (INCI: Sodium Laureth Sulfate) | 32,00% |
| Synthesebeispiel 9 | 0,40% |
| Perfume | 0,50% |
| Water | ad 100% |
| TEGO^{®} Betain F 50, Evonik Industries, 38%-ig (INCI: Cocamidopropyl Betaine) | 8,00% |
| ANTIL^{®} 171, Evonik Industries (INCI: PEG-18 Glyceryl Oleate/Cocoate) | 1,00% |
| NaCl | 0,50% |
| Preservative | q.s. |

**Formulierungsbeispiel 2) Shampoo, PEG- & sulfate-free**

| | |
|---|---|
| REWOTERIC^{®} AM C, Evonik Industries, 32%-ig (INCI: Sodium Cocoamphoacetate) | 15,00% |
| Plantapon ACG 50, BASF (INCI: Disodium Cocoyl Glutamate) | 3,80% |
| Synthesebeispiel 9 | 1,00% |
| Perfume | 0,30% |
| Water | ad 100% |
| TEGO^{®} Betain F 50, Evonik Industries, 38%-ig (INCI: Cocamidopropyl Betaine) | 10,00% |
| VARISOFT^{®} PATC, Evonik Industries (INCI: Palmitamidopropyltrimonium Chloride) | 2,30% |
| ANTIL^{®} SPA 80, Evonik Industries (INCI: Isostearamide MIPA; Glyceryl Laurate) | 2,00% |
| Preservative | 0,30% |
| Citric Acid, 30 %-ig | q.s. |

**Formulierungsbeispiel 3) Conditioning Shampoo**

| | |
|---|---|
| TEXAPON^{®} NSO, BASF, 28%-ig (INCI: Sodium Laureth Sulfate) | 32,00% |
| ANTIL^{®} 200, Evonik Industries (INCI: PEG-200 Hydrogenated Glyceryl Palmate; PEG-7 Glyceryl Cocoate) | 2,00% |
| Synthesebeispiel 9 | 0,50% |
| Perfume | 0,25% |
| Water | ad 100% |
| Polymer JR 400, Amerchol (INCI: Polyquaternium-10) | 0,20% |
| TEGO^{®} Betain F 50, Evonik Industries, 38%-ig (INCI: Cocamidopropyl Betaine) | 8,00% |
| NaCl | 0,30% |
| Preservative | q.s. |

**Formulierungsbeispiel 4) Conditioning Shampoo**

| | |
|---|---|
| TEXAPON^{®} NSO, BASF, 28%-ig (INCI: Sodium Laureth Sulfate) | 32,00% |
| ANTIL^{®} 200, Evonik Industries (INCI: PEG-200 Hydrogenated Glyceryl Palmate; PEG-7 Glyceryl Cocoate) | 2,00% |
| ABIL^{®} Quat 3272, Evonik Industries (INCI: Quaternium-80) | 0,75% |
| Synthesebeispiel 9 | 1,50% |
| Synthesebeispiel 2 | 0,40% |
| Perfume | 0,25% |
| Water | ad 100% |
| Jaguar C-162, Rhodia (INCI: Hydroxypropyl Guar Hydroxypropyltrimonium Chloride) | 0,10% |
| Polymer JR 400, Amerchol (INCI: Polyquaternium-10) | 0,20% |
| REWOTERIC^{®} AM C, Evonik Industries, 32%-ig (INCI: Sodium Cocoamphoacetate) | 3,00% |
| TEGO^{®} Betain F 50, Evonik Industries, 38%-ig (INCI: Cocamidopropyl Betaine) | 5,00% |
| TEGO^{®} Pearl N 300 Evonik Industries (INCI: Glycol Distearate; Laureth-4; Cocamidopropyl Betaine) | 2,00% |
| NaCl | 0,30% |
| Preservative | q.s. |

**Formulierungsbeispiel 5) Shampoo, PEG- & sulfate-free**

| | | |
|---|---|---|
| A | REWOTERIC^{®} AM C, Evonik Industries, 32%-ig (INCI: Sodium Cocoamphoacetate) | 20,00% |
| | REWOPOL^{®} SB F 12 P, Evonik Goldschmidt, 96%-ig (INCI: Disodium Lauryl Sulfosuccinate) | 5,90% |
| | Synthesebeispiel 2 | 1,00% |
| | ANTIL^{®} SPA 80, Evonik Industries, (INCI: Isostearamide MIPA; Glyceryl Laurate) | 1,70% |
| B | Water | ad 100% |
| | Citric Acid, 30%-ig | 3,60% |
| C | ANTIL^{®} HS 60, Evonik Industries, (INCI: Cocamidopropyl Betaine; Glyceryl Laurate) | 3,00% |
| | Preservative | 0,60% |

**Formulierungsbeispiel 6) Rinse-Off Conditioner**

| | |
|---|---|
| Water | ad 100% |
| VARISOFT^{®} EQ 65, Evonik Industries (INCI: Distearoylethyl Dimonium Chloride; Cetearyl Alcohol) | 2,00% |
| VARISOFT^{®} BT 85, Evonik Industries (INCI: Behentrimonium Chloride) | 1,00% |
| Synthesebeispiel 9 | 1,10% |
| TEGO^{®} Alkanol 1618, Evonik Industries (INCI: Cetearyl Alcohol) | 5,00% |
| Preservative, Perfume | q.s. |

**Formulierungsbeispiel 7) Rinse-Off Conditioner**

| | |
|---|---|
| Water | ad 100% |
| VARISOFT^{®} EQ 65, Evonik Industries (INCI: Distearoylethyl Dimonium Chloride; Cetearyl Alcohol) | 2,00% |
| VARISOFT^{®} BT 85, Evonik Industries (INCI: Behentrimonium Chloride) | 2,00% |
| ABIL^{®} Quat 3272, Evonik Industries (INCI: Quaternium-80) | 0,50% |
| Synthesebeispiel 2 | 1,30% |
| Synthesebeispiel 9 | 0,50% |
| TEGO^{®} Alkanol 1618, Evonik Industries (INCI: Cetearyl Alcohol) | 5,00% |
| Preservative, Perfume | q.s. |

**Formulierungsbeispiel 8) Rinse-Off Conditioner**

| | |
|---|---|
| TEGINACID^{®} C, Evonik Industries (INCI: Ceteareth-25) | 0,50% |
| TEGO^{®} Alkanol 16, Evonik Industries (INCI: Cetyl Alcohol) | 2,00% |
| TEGO^{®} Amid S 18, Evonik Industries (INCI: Stearamidopropyl Dimethylamine) | 1,00% |
| Synthesebeispiel 2 | 0,50% |
| Propylene Glycol | 2,00% |
| Citric Acid Monohydrate | 0,30% |
| Water | ad 100% |
| Preservative, Perfume | q.s. |

**Formulierungsbeispiel 9) Leave-In Conditioner Spray**

| | |
|---|---|
| Lactic Acid, 80% | 0,40% |
| Water | ad 100% |
| TEGO^{®} Amid S 18, Evonik Industries (INCI: Stearamidopropyl Dimethylamine) | 1,20% |
| TEGIN^{®} G 1100 Pellets, Evonik Industries (INCI: Glycol Distearate) | 0,60% |
| TEGO^{®} Care PS, Evonik Industries (INCI: Methyl Glucose Sesquistearate) | 1,20% |
| TEGOSOFT^{®} DEC, Evonik Industries (INCI: Diethylhexyl Carbonate) | 0,30% |
| Synthesebeispiel 9 | 1,20% |
| Preservative, Perfume | q.s. |

**Formulierungsbeispiel 10) Leave-In Conditioner Spray**

| | |
|---|---|
| TAGAT^{®} CH 40, Evonik Industries (INCI: PEG-40 Hydrogenated Castor Oil) | 6,00% |
| Ceramide VI, Evonik Industries (INCI: Ceramide 6 II) | 0,05% |
| Perfume | 0,20% |
| Water | ad 100% |
| Synthesebeispiel 2 | 2,00% |
| LACTIL^{®}, Evonik Industries (INCI: Sodium Lactate; Sodium PCA; Glycine; Fructose; Urea; Niacinamide; Inositol; Sodium benzoate; Lactic Acid) | 2,00% |
| TEGO^{®} Betain F 50, Evonik Industries 38% (INCI: Cocamidopropyl Betaine) | 4,30% |
| Citric Acid (10% in water) | 2,00% |

**Formulierungsbeispiel 11) Leave-In Conditioner Foam**

| | |
|---|---|
| Synthesebeispiel 2 | 0,50% |
| TAGAT^{®} CH 40, Evonik Industries (INCI: PEG-40 Hydrogenated Castor Oil) | 0,90% |
| Perfume | 0,30% |
| TEGO^{®} Betain 810, Evonik Industries (INCI: Capryl/Capramidopropyl Betaine) | 2,00% |
| Water | ad 100% |
| TEGO^{®} Cosmo C 100, Evonik Industries (INCI: Creatine) | 0,50% |
| TEGOCEL^{®} HPM 50, Evonik Industries (INCI: Hydroxypropyl Methylcellulose) | 0,30% |
| VARISOFT^{®} 300, Evonik Industries (INCI: Cetrimonium Chloride) | 1,30% |
| LACTIL^{®} Evonik Industries (INCI: Sodium Lactate; Sodium PCA; Glycine; Fructose; Urea; Niacinamide; Inositol; Sodium benzoate; Lactic Acid) | 0,50% |
| Citric Acid (30% in water) | 0,10% |
| Preservative | q.s. |

**Formulierungsbeispiel 12) Strong Hold Styling Gel**

| | |
|---|---|
| TEGO^{®} Carbomer 141, Evonik Industries (INCI: Carbomer) | 1,20% |
| Water | ad 100% |
| NaOH, 25% | 2,70% |
| PVP/VA W-735, ISP (INCI: PVP/VA Copolymer) | 16,00% |
| Synthesebeispiel 2 | 0,50% |
| Synthesebeispiel 9 | 1,00% |
| Alcohol Denat. | 10,00% |
| TAGAT^{®} O 2 V, Evonik Industries (INCI: PEG-20 Glyceryl Oleate) | 2,00% |
| Perfume | 0,30% |
| ABIL^{®} B 88183, Evonik Industries (INCI: PEG/PPG-20/6 Dimethicone) | 0,30% |
| Preservative | q.s. |

**Formulierungsbeispiel 13) Schaumiges Körperpflegemittel**

| | |
|---|---|
| TEXAPON^{®} NSO, BASF, 28%-ig (INCI: Sodium Laureth Sulfate) | 14,30% |
| Perfume | 0,30% |
| Synthesebeispiel 2 | 1,00% |
| REWOTERIC^{®} AM C, Evonik Industries, 32%-ig (INCI: Sodium Cocoamphoacetate) | 8,00% |
| Water | ad 100% |
| Polyquaternium-7, Nalco, (INCI: Merquat 550) | 0,30% |
| LACTIL^{®}, Evonik Industries (INCI: Sodium Lactate; Sodium PCA; Glycine; Fructose; Urea; Niacinamide; Inositol; Sodium benzoate; Lactic Acid) | 0,50% |
| Citric Acid Monohydrate | 0,50% |

**Formulierungsbeispiel 14) Mild Foam Bath**

| | |
|---|---|
| TEXAPON^{®} NSO, BASF, 28%-ig (INCI: Sodium Laureth Sulfate) | 27,00% |
| REWOPOL^{®} SB FA 30, Evonik Industries, 40%-ig (INCI: Disodium Laureth Sulfosuccinate) | 12,00% |
| TEGOSOFT® LSE 65 K SOFT, Evonik Industries (INCI: Sucrose Cocoate) | 2,00% |
| Water | ad 100% |
| REWOTERIC^{®} AM C, Evonik Industries, 32%-ig (INCI: Sodium Cocoamphoacetate) | 13,00% |
| Synthesebeispiel 9 | 0,30% |
| Citric Acid (30% in water) | 3,00% |
| ANTIL^{®} 171, Evonik Industries (INCI: PEG-18 Glyceryl Oleate/Cocoate) | 1,50% |
| TEGO^{®} Pearl N 300 Evonik Industries (INCI: Glycol Distearate; Laureth-4; Cocamidopropyl Betaine) | 2,00% |

**Formulierungsbeispiel 15) Rinse-Off Conditioner**

| | |
|---|---|
| Water | ad 100% |
| VARISOFT^{®} 300, Evonik Industries (INCI: Cetrimonium Chloride) | 2,00% |
| VARISOFT^{®} BT 85, Evonik Industries (INCI: Behentrimonium Chloride) | 2,00% |
| ABIL^{®} OSW 5, Evonik Industries (INCI: Cyclopentasiloxane; Dimethiconol) | 1,00% |
| Synthesebeispiel 2 | 0,80% |
| TEGO^{®} Alkanol 1618, Evonik Industries (INCI: Cetearyl Alcohol) | 5,00% |
| Preservative, Perfume | q.s. |

**Formulierungsbeispiel 16) Rinse-Off Conditioner**

| | |
|---|---|
| Water | ad 100% |
| VARISOFT^{®} BT 85, Evonik Industries (INCI: Behentrimonium Chloride) | 3,00% |
| SF 1708, Momentive (INCI: Amodimethicone) | 2,00% |
| Synthesebeispiel 9 | 0,50% |
| Synthesebeispiel 2 | 0,80% |
| TEGO^{®} Alkanol 1618, Evonik Industries (INCI: Cetearyl Alcohol) | 5,00% |
| Preservative, Perfume | q.s. |

**Formulierungsbeispiel 17, feuchtigkeitsspendendes Hautreinigungsmittel**

| | | |
|---|---|---|
| A | TEXAPON^{®} NSO, BASF, 28%-ig (INCI: Sodium Laureth Sulfate) | 30,00% |
| | Synthesebeispiel 2 | 0,70% |
| | Perfume | 0,30% |
| B | Water | ad 100% |
| | TEGOCEL^{®} fluid HPM 4000, Evonik Industries (INCI: Hydroxypropyl Methylcellulose) | 1,20% |
| | TEGO^{®} Betain C 60, Evonik Industries, 46%-ig (INCI: Cocamidopropyl Betaine) | 8,10% |
| | TEGOSOFT^{®} APM, Evonik Industries (INCI: PPG-3 Myristyl Ether) | 1,00% |
| | Cutina TS, BASF (INCI: PEG- 3 Distearate) | 1,00% |
| | REWODERM^{®} LI S 80, Evonik Industries (INCI: PEG-200 Hydrogenated Glyceryl Palmate; PEG-7 Glyceryl Cocoate) | 1,50% |
| | Preservative | 0,60% |
| | Citric Acid, 30%-ig | q.s. |

**Formulierungsbeispiel 18, Turbid Conditioning Shampoo**

| | |
|---|---|
| TEXAPON^{®} NSO, BASF, 28%-ig (INCI: Sodium Laureth Sulfate) | 32,00% |
| ANTIL^{®} 200, Evonik Industries (INCI: PEG-200 Hydrogenated Glyceryl Palmate; PEG-7 Glyceryl Cocoate) | 2,00% |
| Synthesebeispiel 2 | 1,00% |
| Perfume | 0,25% |
| Water | ad 100% |
| Polymer JR 400, Amerchol (INCI: Polyquaternium-10) | 0,20% |
| TEGO^{®} Betain F 50, Evonik Industries, 38%-ig (INCI: Cocamidopropyl Betaine) | 8,00% |
| DC1503 Fluid, Dow Corning (INCI: Dimethicone; Dimethiconol) | 1,00% |
| TEGO^{®} Pearl N 300 Evonik Industries (INCI: Glycol Distearate; Laureth-4; Cocamidopropyl Betaine) | 2,00% |
| NaCl | 0,30% |
| Preservative | q.s. |

**Formulierungsbeispiel 19) Mild Hair & Body Wash, PEG- und Sulfate-free**

| | |
|---|---|
| Plantacare^{®} 1200 UP, BASF, 50%-ig (INCI: Lauryl Glucoside) | 11,40% |
| Plantacare^{®} 818 UP, BASF, 51%-ig (INCI: Coco Glucoside) | 5,60% |
| Water | ad 100% |
| ANTIL^{®} Soft SC, Evonik Industries (INCI: Sorbitan Sesquicaprylate) | 0,90% |
| Synthesebeispiel 2 | 1,00% |
| TEGOSOFT^{®} LSE 65 K SOFT, Evonik Industries (INCI: Sucrose Cocoate) | 1,50% |
| TEGO^{®} Betain F 50, Evonik Industries, 38%-ig (INCI: Cocamidopropyl Betaine) | 18,00% |
| Perfume, preservative | q.s. |
| Citric Acid, 30% | q.s. |

**Formulierungsbeispiel 20) Sprayable Hairmilk, PEG-free**

| | | |
|---|---|---|
| A | Water | ad 100% |
| | Lactic Acid, 80%-ig | 0,40% |
| B | TEGO^{®} AMID S 18, Evonik Industries (INCI: Stearamidopropyl Dimethylamine) | 1,20% |
| | TEGIN^{®} G 1100 Pellets, Evonik Industries (INCI: Glycol Distearate) | 0,60% |
| | TEGO^{®} Care PS, Evonik Industries (INCI: Methyl Glucose Sesquistearate) | 1,20% |
| | TEGOSOFT^{®} DEC, Evonik Industries (INCI: Diethylhexyl Carbonate) | 0,30% |
| | Synthesebeispiel 2 | 0,60% |
| | Perfume, preservative | q.s. |

**Formulierungsbeispiel 21: Conditioning Anti-Schuppen Shampoo**

| | | |
|---|---|---|
| A | TEGIN^{®} G 1100 Pellets, Evonik Industries (INCI: Glycol Distearate) | 3,00% |
| | TEXAPON^{®} NSO, Cognis, 28%-ig (INCI: Sodium Laureth Sulfate) | 40,00% |
| B | Perfume | 0,30% |
| | Zinc-Pyrion NF, WeylChem, 48%-ig (INCI: Zinc Pyrithione) | 2,00% |
| | Synthesebeispiel 9 | 1,00% |
| C | Water | ad 100% |
| | TEGO^{®} Carbomer 341 ER, Evonik Industries (INCI: Acrylates/C10-30 Alkyl Acrylate Crosspolymer) | 0,20% |
| | Water | 0,30% |
| | NaOH, 25%-ig | 0,30% |
| D | REWOTERIC^{®} AM B U 185, Evonik Industries, 30%-ig (INCI: Undecylenamidopropyl Betaine) | 12,50% |
| | ANTIL^{®} SPA 80, Evonik Industries (INCI: Isostearamide MIPA; Glyceryl Laurate) | 3,70% |
| E | Preservative | q.s. |

**Formulierungsbeispiel 22: Haarfärbemittel**

| | |
|---|---|
| Water demineralized | ad 100% |
| TEGO^{®} Alkanol 1618, Evonik Industries, (INCI: Cetearyl Alcohol) | 12,00% |
| Eutanol^{®} G, BASF (INCI: Octyldodecanol) | 3,00% |
| REWOMID^{®} C 212, Evonik Industries (INCI: Cocamide MEA) | 1,50% |
| Super Hartolan^{®} B, Crodo (INCI: Lanolin Alcohol) | 3,00% |
| Avocadoöl, Henry Lamotte (INCI: Persea Gratissima Oil) | 1,50% |
| Pristerene^{®} 4960, Uniquema (INCI: Stearic Acid) | 6,00% |
| EDTA BD, BASF (INCI: Disodium EDTA) | 0,10% |
| Texapon^{®} K12G, BASF (INCI: Sodium Lauryl Sulfate) | 0,50% |
| Propylenglycol | 5,00% |
| Timica Silver Sparkle, BASF (INCI: MICA; Titanium Dioxide) | 1,00% |
| Ammoniaklösung, 25%ig | 6,00% |
| 2,5-Diaminotoluolsulfat, (INCI: Toluene-2,5-Diamine) | 1,40% |
| Rodol^{®} RS, Jos. H. Lowenstein & Sons (INCI: Resorcinol) | 0,30% |
| HC Blue A42, (INCI: 2,4-Diaminophenoxyethanol di HCl) | 0,10% |
| Natriumsulfit | 0,50% |
| Perfume | 0,20% |
| Synthesebeispiel 9 | 0,50% |

**Formulierungsbeispiel 23: Shampoo**

| | |
|---|---|
| TEXAPON^{®} NSO, Cognis, 28%-ig (INCI: Sodium Laureth Sulfate) | 28,00% |
| REWOTERIC^{®} AM 2 C NM, Evonik Industries, 39%-ig (INCI: Disodium Cocoamphodiacetate) | 4,00% |
| TEGO^{®} Betain F 50, Evonik Industries, 38%-ig (INCI: Cocamidopropyl Betaine) | 7,00% |
| REWOMID^{®} C 212, Evonik Industries (INCI: Cocamide MEA) | 0,80% |
| ANTIL^{®} 171, Evonik Industries (INCI: PEG-18 Glyceryl Oleate/Cocoate) | 0,50% |
| N-Hance^{®} SP-100, Hercules (INCI: Acrylamidopropyl Trimonium Chloride/Acrylamide Copolymer) | |
| Polymer JR 400, Amerchol (INCI: Polyquaternium-10) | 0,10% |
| Jaguar C-162, Rhodia (INCI: Hydroxypropyl Guar Hydroxypropyltrimonium Chloride) | 0,20% |
| DC 193, Dow Corning (INCI: PEG-12 Dimethicone) | 0,40% |
| Synthesebeispiel 2 | 0,70% |
| Synthesebeispiel 9 | 0,60% |
| TEGIN^{®} D 1102, Evonik Industries (INCI: PEG-3 Distearate) | 0,40% |
| TAGAT^{®} CH 40, Evonik Industries (INCI: PEG-40 Hydrogenated Castor Oil) | 0,20% |
| Water | ad 100% |
| NaCl | 0,70% |
| Citric Acid | ad. pH=~5,5 |
| Perfume | q.s. |
| Preservative | q.s. |

## Patentansprüche

1. Siloxan-Polymer der allgemeinen Formel I umfassend einen zentralen Polysiloxan-Polymerblock B,
(i.) der mit organofunktionellen Resten substituiert ist,
(ii) der Polymerblock B weist lineare und/oder verzweigte Strukturen mit mindestens zwei difunktionellen Siloxan-Einheiten auf,
(iii.) der Polymerblock B weist an mindestens zwei terminalen Silizium-Atomen oder mindestens einem terminalen und mindestens einem seitenkettenständigen Silizium-Atom der Siloxan-Einheiten des Polymerblocks B die organofunktionellen Reste -Q1 und -Q2 auf, wobei Reste gleich oder verschieden sind,
Q2-B-Q1 (I)
wobei -Q1 der allgemeinen Formel IIa und -Q2 der Formel IIb entsprechen,
-Q1 =-Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
- mit A gleich -NH-, -O- oder -S- und D gleich -NH- jeweils unabhängig in Formeln IIa und IIb,
- mit A' gleich -NH- und D' gleich -NH-, -O- oder -S- jeweils unabhängig in Formeln IIa und IIb, wobei jeder Rest Q1 und Q2 der Formeln IIa oder IIb jeweils unabhängig mindestens zwei bivalente Gruppen ausgewählt aus Carbamat- und Harnstoff- Gruppe aufweist,
- mit Q1' und Q2'jeweils unabhängig umfassend einen bivalenten Kohlenwasserstoff-Rest mit 6 bis 200 C-Atomen optional umfassend mindestens ein Heteroatom ausgewählt aus O, N und S, einen bivalenten Rest umfassend Aryl-, Arylalkyl-Gruppen optional umfassend mindestens ein Heteroatom O, N oder S oder Alkyl-, Aryl- oder Alkyl- und Aryl-Gruppen enthaltende Polyether-Reste,
- mit Q1" und Q2" jeweils unabhängig umfassend einen bivalenten linearen, verzweigten und/oder cyclischen Alkyl-Rest mit 4 bis 200 C-Atomen oder einen bivalenten Rest umfassend einen Aryl- und/oder Arylalkyl-Rest mit 6 bis 200 C-Atomen, und
- mit -D'-Q1* und -D'-Q2* mit D', wie vorstehend definiert, wobei -D'-Q1* und -D'-Q2* jeweils unabhängig als Reste Q1* und Q2* ein UV/Vis-Chromophor als Rest umfassen.

2. Siloxan-Polymer nach Anspruch 1,
**dadurch gekennzeichnet,**
**dass** in dem Siloxan-Polymer der allgemeinen Formeln I, IIa und IIb -D'-Q1* und -D'-Q2* jeweils unabhängig als Reste ein UV-Chromophor umfassen mit mindestens einem Absorptionsmaximum im Bereich von 280 bis 380 nm, besonders bevorzugt mit mindestens einem Absorptionsmaximum im Spektralbereich von 300 bis 380 nm.

3. Siloxan-Polymer nach Anspruch 1 oder 2,
**dadurch gekennzeichnet,**
**dass** in dem Siloxan-Polymer der allgemeinen Formeln I, IIa und IIb -D'-Q1 * und -D'-Q2* jeweils unabhängig ein Vis-Chromophor als Rest umfassen mit mindestens einem Absorptionsmaximum im Bereich von 320 bis 790 nm, insbesondere 320 bis 380 nm.

4. Siloxan-Polymer nach einem der Ansprüche 1 bis 4,
**dadurch gekennzeichnet,**
**dass** in dem Siloxan-Polymer der allgemeinen Formel I der Polymerblock B der allgemeinen Formel IIIa oder IIIb entspricht, mit B gleich 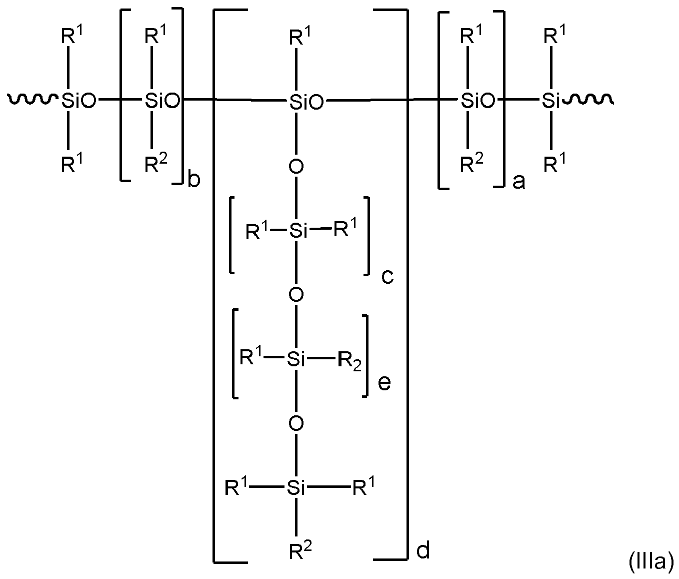 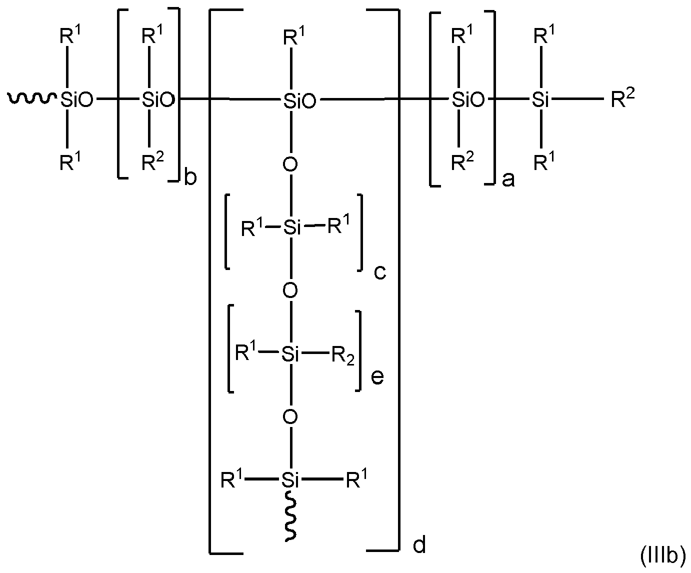 mit a, b, c, d und e in Formeln IIIa und IIIb jeweils unabhängig eine ganze Zahl mit a von 1 bis 200, mit b von 0 bis 200, mit c von 0 bis 200, mit d von 0 bis 200, mit e von 0 bis 200 und mit R¹ in Formeln IIIa oder IIIb jeweils unabhängig gleich oder verschieden, wobei R¹ umfasst Alkyl-Reste mit 1 bis 22 C-Atomen, vorzugsweise 1 bis 4 C-Atomen oder Phenyl-Reste, mit R² in Formeln IIIa oder IIIb gleich Alkyl-Rest mit 1 bis 22 C-Atomen, vorzugsweise 1 bis 4 C-Atomen, ein Alkyl-Rest mit mindestens einem Heteroatom ausgewählt aus N, O, S oder Phenyl-Rest.

5. Siloxan-Polymer nach einem der Ansprüche 1 bis 3,
**dadurch gekennzeichnet,**
**dass** b, c, d und e gleich 0 sind und a gleich 20 bis 100 ist, insbesondere 30 oder 80 mit einer Schwankungsbreite von plus/minus 5.

6. Siloxan-Polymer nach einem der Ansprüche 1 bis 4,
**dadurch gekennzeichnet,**
**dass** R¹ und R² ausgewählt sind aus Alkyl-Gruppen mit 1, 2, 3 oder 4 C-Atomen, insbesondere aus Methyl-Gruppen oder mindestens ein R² eine Aminoalkyl-Gruppe, insbesondere mit einer primären Amino-Gruppe oder eine quartäre Alkylamin-Gruppe ist.

7. Siloxan-Polymer nach einem der Ansprüche 1 bis 5,
**dadurch gekennzeichnet,**
**dass** die Reste -Q1 und -Q2 in der allgemeinen Formel I unabhängig ausgewählt sind aus
-Q1 =-Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
a) mit A gleich -O-, D gleich -NH-, A' gleich -NH- und D' gleich -O-,
b) mit A gleich -O-, D gleich -NH-, A' gleich -NH- und D' gleich -NH-,
c) mit A gleich -NH-, D gleich -NH-, A' gleich -NH- und D' gleich -NH-,
d) mit A gleich -S-, D gleich -NH-, A' gleich -NH- und D' gleich -NH-,
e) mit A gleich -NH-, D gleich -NH-, A' gleich -NH- und D' gleich -O- oder
f) mit A gleich -S-, D gleich -NH-, A' gleich -NH- und D' gleich -O-,
g) mit A gleich -O-, D gleich -NH-, A' gleich -NH- und D' gleich -S-,
h) mit A gleich -NH-, D gleich -NH-, A' gleich -NH- und D' gleich -S- oder
i) mit A gleich -S-, D gleich -NH-, mit A' gleich -NH- und D' gleich -S-.

8. Siloxan-Polymer nach einem der Ansprüche 1 bis 6,
**dadurch gekennzeichnet,**
**dass** in den Resten -Q1 und -Q2 der Formel I die Reste -D'-Q1* und -D'-Q2* jeweils unabhängig abgeleitet sind aus einem Hydroxycumarin oder Hydroxycumarin-Derivat, insbesondere 7-Hydroxycumarin, 4-Hydroxycumarin, 6-Hydroxycumarin, Hydroxy-Isocumarin, ein Amino-Cumarin oder Amino-Cumarin-Derviat.

9. Siloxan-Polymer nach einem der Ansprüche 1 bis 7,
**dadurch gekennzeichnet,**
**dass** in den Resten -Q1 und -Q2 der Formel I
A gleich -O-, D gleich -NH-, A' gleich -NH- und D' gleich -O- sind, und die Reste -D'-Q1* und -D'-Q2* jeweils unabhängig abgeleitet sind ausgewählt aus einem Hydroxycumarin oder Hydroxycumarin-Derivat, insbesondere 7-Hydroxycumarin, 4-Hydroxycumarin und 6-Hydroxycumarin.

10. Siloxan-Polymer nach einem der Ansprüche 1 bis 7,
**dadurch gekennzeichnet,**
**dass** in dem Siloxan-Polymer der allgemeinen Formeln I, IIa und IIb die Reste ausgewählt aus -D'-Q1 * und -D'-Q2* jeweils unabhängig mit D' gleiche -NH- und mit Q1* oder Q2* ein Amino-funktioneller Kohlenwasserstoff-Rest.

11. Siloxan-Polymer nach einem der Ansprüche 1 bis 10,
**dadurch gekennzeichnet,**
**dass** in den Resten -Q1 und -Q2 der Formel I die bivalenten Reste -Q1 "- und -Q2"- unabhängig ausgewählt sind aus bivalenten, linearen, verzweigten oder cyclischen Alkylen-Resten mit 4 bis 25 C-Atomen, insbesondere mit 6, 7, 8, 9, 10, 11, 12, 13 oder 14 C-Atomen, vorzugsweise Hexylen (-CH₂)₆, Heptylen, bivalentes 2,4-Toluolyl, Diphenylmethan, polymeres Diphenylmethan, 3,5,5-Trimethyl-1-methylen-3-ethylen-cyclohexan oder 4,4'-Dicyclohexylen.

12. Siloxan-Polymer nach einem der Ansprüche 1 bis 11,
**dadurch gekennzeichnet,**
**dass** in den Resten -Q1 und -Q2 der Formel I die bivalenten Reste -Q1'- und -Q2'-ausgewählt sind aus Alkylen-Resten mit 3 bis 22 C-Atomen optional mit mindestens einem Heteroatom umfassend N, O oder S, insbesondere -(CH₂)ₙ mit n von 3 bis 22 C-Atomen, vorzugsweise Hexylen (-CH₂)₆-, Heptylen (-CH₂)₇-, Octylen (-CH₂)₈-, Nonylen (-CH₂)₉-, Decylen (-CH₂)₁₀-, Undecylen (-CH₂)₁₁-, Dodecylen (-CH₂)₁₂-, oder mit Heteroatomen, Alkylen-(C=O)-, Alkylen-O(CO)-Alkylen, Alkylen-(CO)O-Alkylen, Alkylen-NH(CO)-Alkylen, Alkylen-(CO)NH-Alkylen, Alkylen-NH(CO)NH-Alkylen, Alkylen-NH(CO)O-Alkylen, oder aus Alkyl-, Aryl- oder Alkyl- und Aryl-Gruppen enthaltenden Polyether-Resten den Formeln IVa oder IVb mit Q1' und Q2' jeweils unabhängig gleich
-T-O-(CH₂-CH₂-O-)ₓ-(CH₂-CH(R^{#})O-)_{y}-(SO)-R" (IVa)
-T-O-(CH₂-CH₂-O-)ₓ-(CH₂-CH(R^{#})O-)_{y}-R" (IVb),
mit T = bivalenter Kohlenwasserstoffrest mit 2 bis 4 C-Atomen, mit x = 0 bis 200, y = 0 bis 200, wobei x und y ganze Zahlen sind, mit der Maßgabe, dass x oder y mindestens 1 ist, mit R^{#} Wasserstoff oder Methyl, R" Alkylen, insbesondere Ethylen, vorzugsweise mit T = -(CH₂)₂- oder -(CH₂)₃-.

13. Siloxan-Polymer nach einem der Ansprüche 1 bis 12,
**dadurch gekennzeichnet,**
**dass** in den Resten -Q1 und -Q2 der Formel I mindestens einer der bivalenten Reste -Q1 "- und -Q2"- unabhängig ein bivalenter Cyclohexan enthaltender-Rest ausgewählt aus den Formeln Va und Vb 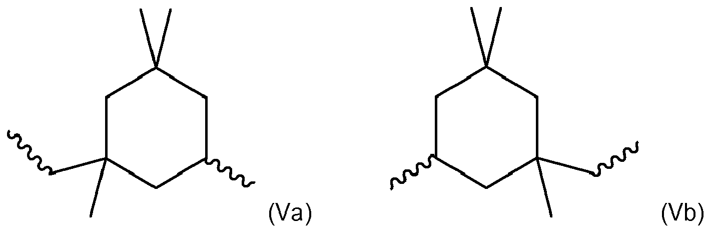 ist, insbesondere ist Q2"- ein bivalenter Cyclohexan enthaltender-Rest der Formel Va und -Q1" der Formel Vb.

14. Siloxan-Polymer nach einem der Ansprüche 1 oder 2 und 7 bis 13, **dadurch gekennzeichnet,**
**dass** Siloxan der allgemeinen Formel I dem Siloxan-Polymer der allgemeinen Formel XI entspricht mit
Mₐ₁M^{A}ₐ₂M^{B}ₐ₃D_{b1}D^{A}_{b2}D^{B}_{b3}T_{c1}T^{A}c2T^{B}_{c3}Q_{d1} (XI)
mit M = [R¹⁶₃SiO_{1/2}], M^{A} = [R¹⁷R¹⁶₂SiO_{1/2}], M^{B} = [R¹⁸R¹⁶₂SiO_{1/2}],
D = [R¹⁶₂SiO_{2/2}], D^{A} = [R¹⁷₁R¹⁶₁SiO_{2/2}], D^{B} = [R¹⁸₁R¹⁶₁Si0_{2/2}],
T = [R¹⁶SiO_{3/2}], T^{A} = [R¹⁷SiO_{3/2}], T^{B}=[R¹⁸SiO_{3/2}], Q = [SiO_{4/2}],
mit
R¹⁶ unabhängig voneinander gleiche oder verschiedene lineare oder verzweigte, gesättigte oder ungesättigte Kohlenwasserstoffreste mit 1 bis 30 Kohlenstoffatomen oder auch aromatische Kohlenwasserstoffreste mit 6 bis 30 Kohlenstoffatomen bevorzugt Methyl oder Phenyl, insbesondere Methyl,
R¹⁷ ist jeweils unabhängig -Q1 oder -Q2,
R¹⁸ unabhängig voneinander gleiche oder verschiedene lineare oder verzweigte, gesättigte oder olefinisch ungesättigte Kohlenwasserstoffreste mit 8 bis 30 Kohlenstoffatomen, aromatischer Kohlenwasserstoffrest mit 6 bis 40 Kohlenstoffatomen, Alkylarylrest mit 7 bis 40 Kohlenstoffatomen, ein durch ein oder mehrere Heteroatome, wie Sauerstoff, NH, NR' mit R' gleich einem gegebenenfalls Doppelbindungen enthaltenden C1 bis C30-Alkylrest unterbrochenen linearen oder verzweigten gegebenenfalls Doppelbindungen enthaltenden aliphatischen Kohlenwasserstoffrest mit 2 bis 30 Kohlenstoffatomen, ein durch eine oder mehrere Funktionalitäten, ausgewählt aus der Gruppe -OH, -O-C(O)-, -(O)C-O-, -NH-C(O)-, -(O)C-NH, - (CH₃)N-C (O)-, -(O)C-N(CH₃)-, -S(O₂)-O-, -O-S(O₂)-, -S(O₂)-NH-, -NH-S(O₂)-, -S (O₂)-N (CH₃)-, -N(CH₃)-S(O₂)-, unterbrochenen linearen oder verzweigten gegebenenfalls Doppelbindungen enthaltenden aliphatischen Kohlenwasserstoffrest mit 2 bis 30 Kohlenstoffatomen, ein endständig OH, OR', NH², N(H)R', N(R')₂ mit R' gleich einem gegebenenfalls Doppelbindungen enthaltenden C1- bis C30-Alkylrest, funktionalisierten linearen oder verzweigten gegebenenfalls Doppelbindungen enthaltenden aliphatischen oder cycloaliphatischen Kohlenwasserstoffrest mit 1 bis 30 Kohlenstoffatomen oder einen blockweise oder statistisch aufgebauten Polyether gemäß -(R⁵-O)ₙ-R⁶, wobei R⁵ ein 2 bis 4 Kohlenstoffatome enthaltender linearer oder verzweigter Kohlenwasserstoffrest, n 1 bis 100, vorzugsweise 2 bis 60, ist und R⁶ Wasserstoff, einen linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einen aromatischen Kohlenwasserstoffrest mit 6 bis 40 Kohlenstoffatomen, einen Alkylarylrest mit 7 bis 40 Kohlenstoffatomen, oder ein Rest -C(O)-R⁷ mit R⁷ gleich einem linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einem aromatischen Kohlenwasserstoffrest mit 6 bis 40 Kohlenstoffatomen, einem Alkylarylrest mit 7 bis 40 Kohlenstoffatomen,
mit den Indices
a1 = 0-200, bevorzugt 1-60, insbesondere 0,
a2 = 0-30, bevorzugt 1-20, insbesondere 2-10,
a3= 0-30, bevorzugt 1-20,
b1 = 2 bis 5000, bevorzugt 10 bis 1000,
b2 = 0 bis 100, bevorzugt 1 bis 30, insbesondere 1 bis10 oder 0,
b3 = 0 bis 100, bevorzugt 0 bis 30, insbesondere 1 bis 10 oder 0,
c1 = 0 bis 30, bevorzugt 1 bis 30,
c2 = 0 bis 30, bevorzugt 0 bis 5, insbesondere 0,
c3 = 0 bis 30, bevorzugt 0 bis 5, insbesondere 0,
d1 = 0 bis 30, bevorzugt 0 bis 5, vorzugsweise 0
mit der Maßgabe, dass mindestens einer der Indices ausgewählt aus a2 und a3 ungleich 0 ist, vorzugsweise ist a2 eine ganze Zahl zwischen 2 und 10.

15. Siloxan-Polymer nach einem der Ansprüche 1 bis 14,
**dadurch gekennzeichnet,**
**dass** es ausgewählt ist aus Siloxan-Polymeren der Formeln Ia und Ib oder Mischungen dieser 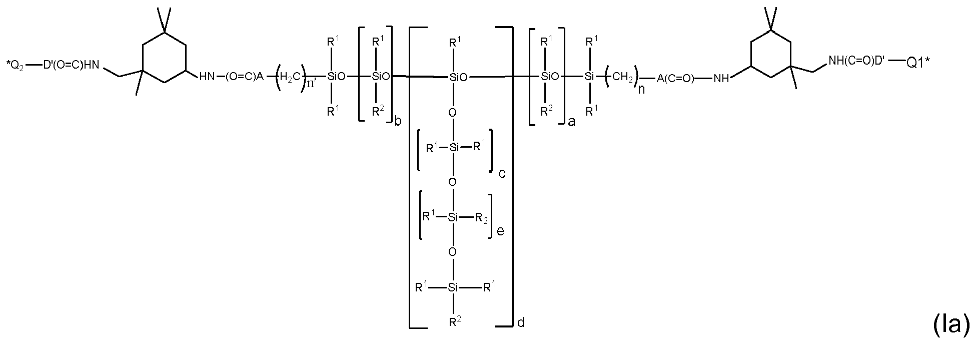 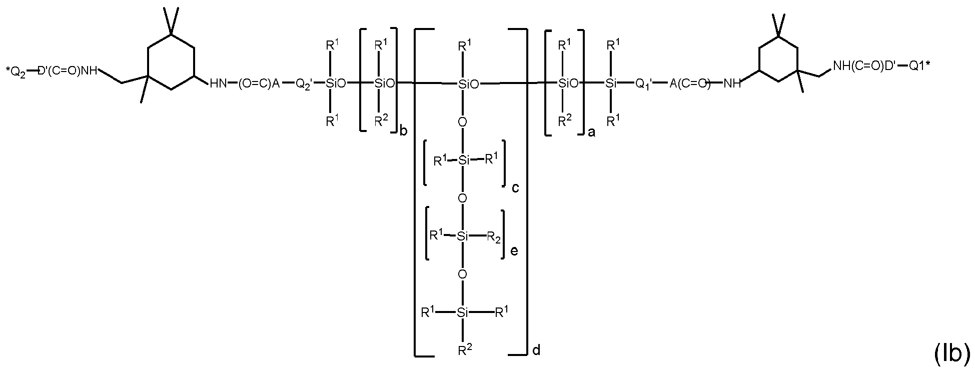 mit n oder n' jeweils unabhängig ausgewählt aus einer ganzen Zahl von 3 bis 22, mit a von 1 bis 200, mit b von 0 bis 200, mit c von 0 bis 200, mit d von 0 bis 200, mit e von 0 bis 200 und mit R¹ in Formeln Ia und Ib jeweils unabhängig gleich oder verschieden, wobei R¹ umfasst Alkyl-Reste mit 1 bis 4 C-Atomen oder Phenyl-Reste, mit R² Alkyl-Rest mit 1 bis 22 C-Atomen, vorzugsweise 1 bis 4 C-Atomen, ein Alkyl-Rest mit mindestens einem Heteroatom ausgewählt aus N, O S oder Phenyl-Rest, insbesondere ist R² ein Alkylamin oder ein durch Reaktion eines Alkylamins mit einem Cumarin-Isocyanat erhaltener Rest, insbesondere der Formel IX, und mit -D'-Q1* und -D'-Q2* jeweils unabhängig abgeleitet aus einem Hydroxy-Cumarin oder Hydroxy-Cumarin-Derivat, insbesondere mit D' = Sauerstoff, und
in Formel Ib mit Q1' und Q2'jeweils unabhängig gleich Alkylen mit 2 bis 40 C-Atomen oder einem Polyether der Formeln IVa oder IVb
-T-O-(CH₂-CH₂-O-)ₓ-(CH₂-CH(R^{#})O-)_{y}-(SO)-R" (IVa)
-T-O-(CH₂-CH₂-O-)ₓ-(CH₂-CH(R^{#})O-)_{y}-R" (IVb)
ist, mit T = bivalenter Kohlenwasserstoffrest mit 2 bis 4 C-Atomen, mit x = 0 bis 200, y = 0 bis 200, wobei x und y ganze Zahlen sind mit der Maßgabe, dass x oder y mindestens 1 ist, mit R⁴ Wasserstoff oder Methyl, R" Alkylen, insbesondere Ethylen, insbesondere mit T = -(CH₂)₂- oder -(CH₂)₃-.

16. Siloxan-Polymer nach einem der Ansprüche 1 bis 15,
**dadurch gekennzeichnet,**
**dass** sie ausgewählt sind aus Siloxan-Polymeren der Formel Ia* und Ib* 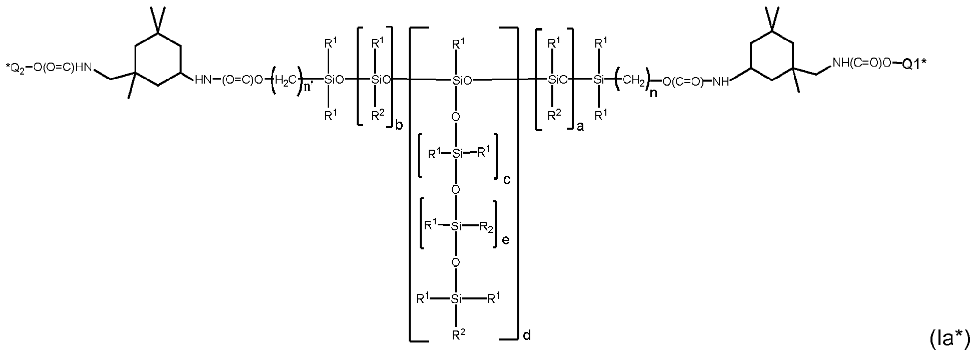 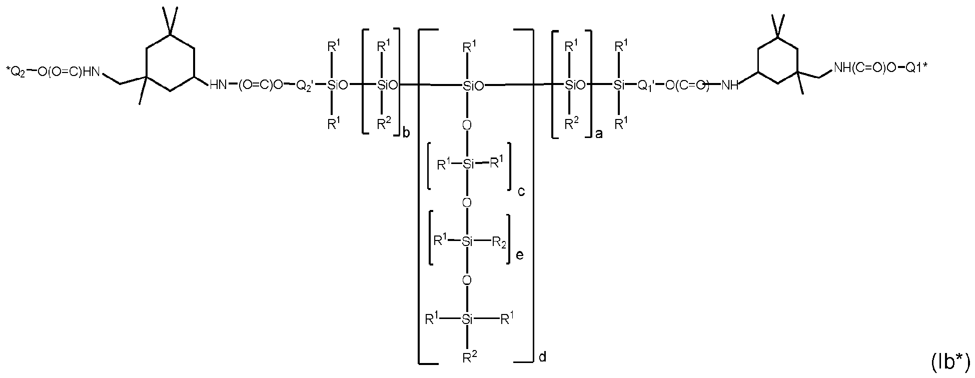 mit n oder n' jeweils unabhängig ausgewählt aus einer ganzen Zahl von 3 bis 22, mit a von 1 bis 200, mit b von 0 bis 200, mit c von 0 bis 200, mit d von 0 bis 200, mit e von 0 bis 200 und
mit R¹ in Formeln Ia* und Ib* jeweils unabhängig gleich oder verschieden, wobei R¹ umfasst Alkyl-Reste mit 1 bis 4 C-Atomen oder Phenyl-Reste, mit R² Alkyl-Rest mit 1 bis 22 C-Atomen, vorzugsweise 1 bis 4 C-Atomen, ein Alkyl-Rest mit mindestens einem Heteroatom umfassend N, O S oder Phenyl-Rest, und
mit Q1 * und Q2* jeweils unabhängig Cumarin oder ein Cumarin-Derivat, insbesondere Cumarin gebunden an C-7, C-4 oder C-6 des Cumarins oder eines Cumarin-Derivates,
und in Formel Ib* mit Q1' und Q2' jeweils unabhängig gleich
-T-O-(CH₂-CH₂-O-)ₓ-(CH₂-CH(R^{#})O-)_{y}-(SO)-R" (IVa)
-T-O-(CH₂-CH₂-O-)ₓ-(CH₂-CH(R^{#})O-)_{y}-R" (IVb),
mit T = bivalenter Kohlenwasserstoffrest mit 2 bis 4 C-Atomen, mit x = 0 bis 200,
y = 0 bis 200, wobei x und y ganze Zahlen sind mit der Maßgabe, dass x oder y mindestens 1 ist, mit R^{#} Wasserstoff oder Methyl, R" Wasserstoff oder Alkylen, insbesondere Ethylen, insbesondere mit T = -(CH₂)₂- oder -(CH₂)₃-.

17. Verfahren zur Herstellung eines Siloxan-Polymers sowie von Zusammensetzungen enthaltend diese Siloxan-Polymere oder Mischungen der Siloxan-Polymere mit einem zentralen Polysiloxan-Polymerblock B, indem
a) ein Polysiloxan-Diisocyanat der Formel VII,
OCN-Q2"-D-(O=C)-A-Q2'-B-Q1'-A-(C=O)-D-Q1 "-NCO (VII)
mit mindestens einem Hydroxy- oder Amino-funktionellen UV/Vis-Chromophor umgesetzt wird und ein Siloxan-Polymer der allgemeinen Formel I
Q2-B-Q1 (I)
erhalten wird, wobei -Q1 der allgemeinen Formel IIa und -Q2 der Formel IIb entsprechen,
-Q1 =-Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
- mit A gleich -NH-, -O- oder -S- und D gleich -NH- jeweils unabhängig in Formeln IIa und IIb,
- mit A' gleich -NH- und D' gleich -NH-, -O- oder -S- jeweils unabhängig in Formeln IIa und IIb, wobei jeder Rest Q1 und Q2 der Formel IIa oder IIb jeweils abhängig mindestens zwei bivalente Gruppen ausgewählt aus Carbamat- und Harnstoff-Gruppe aufweist,
oder
b) ein Polysiloxan der Formel VI
HA-Q2'-B-Q1'-AH (VI)
mit einem UV/Vis-Chromophor-Isocyanat ausgewählt aus den Formeln IXa,IXb, IXc, IXd oder Gemischen enthaltend diese
Q2*-O(CO)NH-"2Q-NCO (IXa) Q1 *-O(CO)NH-"1 Q-NCO (IXb)
Q2*-O(CO)NH-Q2"-NCO (IXc) Q1*-O(CO)NH-Q1"-NCO (IXd)
umgesetzt wird,
- mit A gleich -NH-, -O- oder -S- und D gleich -NH- jeweils unabhängig in Formeln VII, I und VI,
- mit Q1' und Q2' jeweils unabhängig umfassend einen bivalenten Kohlenwasserstoff-Rest mit 6 bis 200 C-Atomen optional umfassend mindestens ein Heteroatom O, N oder S, einen bivalenten Rest umfassend Aryl-, Arylalkyl-Gruppen oder einen bivalenten Rest umfassend Aryl-, Arylalkyl-Gruppen optional umfassend mindestens ein Heteroatom O, N oder S oder Alkyl-, Aryl- oder Alkyl- und Aryl-Gruppen enthaltende Polyether-Reste, jeweils unabhängig in Formeln VII, I und VI,
- mit Q1" und Q2" jeweils unabhängig umfassend einen bivalenten linearen, verzweigten und/oder cyclischen Alkyl-Rest mit 4 bis 200 C-Atomen, oder einen bivalenten Rest umfassend einen Aryl- und/oder Arylalkyl-Rest mit 6 bis 200 C-Atomen, jeweils unabhängig in Formeln VII, I, IXa, IXb, IXc und IXd
- mit -O-Q1* oder -O-Q2* jeweils unabhängig als -Q1* und -Q2* ein UV/Vis-Chromophor als Rest.

18. Verfahren nach Anspruch 17,
**dadurch gekennzeichnet,**
**dass** (i) das Hydroxy- oder Amino-funktionelle UV/Vis-Chromophor ein UV-Chromophor umfassend Hydroxy-Cumarin, Amino-Cumarin oder ein Derivat von Cumarin ist,
(ii) das UV/Vis-Chromophor-Isocyanat ein UV-Chromophor ausgewählt aus Cumarin-Isocyanaten aus den Formeln IXa, IXb, IXc und IXd ist, mit -O-Q1* und -O-Q2* jeweils unabhängig als Rest -Q1 * und/oder -Q2* Cumarin oder Cumarin-Derivat, und/oder
(iii) in Formel IIa, IIb, IXa, IXb, IXc und/oder IXd und I-O-Q1* und -O-Q2* jeweils unabhängig Reste umfassen aus der Umsetzung von Hydroxycumarin oder Hydroxycumarin-Derivat, insbesondere 7-Hydroxycumarin, 4-Hydroxycumarin, 6-Hydroxycumarin oder Hydroxy-Isocumarin mit einem Diisocyanat.

19. Verfahren nach Anspruch 17 oder 18,
**dadurch gekennzeichnet,**
**dass** ein Polysiloxan der Formel VI
HA-Q2'-B-Q1'-AH (VI)
eingesetzt wird, mit A ausgewählt aus -O, -NH bzw. AH ausgewählt aus -OH, -NH₂ oder -SH mit -Q2'- und -Q1'-, wie vorstehend definiert, und umgesetzt wird mit einem Diisocyanat zu einem Polysiloxan-Diisocyanat der Formel VII,
OCN-Q2"-D-(O=C)-A-Q2'-B-Q1'-A-(C=O)-D-Q1 "-NCO (VII)
mit -Q2"- und/oder -Q1 "- unabhängig ausgewählt aus einem bivalenten, linearen, verzweigten und/oder cyclischen Alkyl-Rest mit 4 bis 200 C-Atomen, oder einem bivalenten Rest umfassend einen Aryl- und/oder Arylalkyl-Rest mit 6 bis 200 C-Atomen, wobei das molare Verhältnis von HA-Gruppen im Polysiloxan zu Isocyanat-Gruppen in Formel VII mindestens 1 zu 1 beträgt, insbesondere ist das Verhältnis 1 : 100 bis 1 : 1, vorzugsweise 1 : 10 bis 1 : 1.

20. Verfahren nach einem der Ansprüche 17 bis 19,
**dadurch gekennzeichnet,**
**dass** (i) a) ein Polysiloxan-Gruppen enthaltender linearer und/oder verzweigter Polymerblock B, insbesondere der Formeln IIIa und/oder IIIb, mit mindestens zwei terminalen Si-H-Gruppen oder mindestens einer terminalen Si-H-Gruppen und mindestens einer seitenkettenständigen Si-H-Gruppe, oder b) ein Polysiloxan-Gruppen der Formel XI mit mindestens ein R¹⁷ Wasserstoff, vorzugsweise zwei R¹⁷ sind Wasserstoff,
(ii) mit einer olefinischen Verbindung umfassend Alkylen und optional umfassend mindestens ein Heteroatom N oder O, insbesondere Alkenylenol, Alkylenamin, quartäres Alkylamin, Alkylencarbonsäure, Alkylenester, Alkylenamid oder einen olefinischen Polyether umgesetzt wird,
wobei die olefinische Verbindung jeweils unabhängig eine Allyl- oder Vinyl-Gruppe aufweist und den Formeln VIIIa und/oder Vlllb entspricht
Q1'-AH (VIIIa)
Q2'-AH (Vlllb)
(iii) und die Umsetzung in Gegenwart eines Katalysators zu einem Polysiloxan der Formel VI
HA-Q2'-B-Q1'-AH (VI)
erfolgt, wobei jeweils unabhängig in Formeln VIIIa, Vlllb und VI mit AH unabhängig ausgewählt aus -OH und -NH₂, und mit -Q2'- und -Q1'- jeweils unabhängig in Formeln VIIIa, VIIIb und VI umfassend einen bivalenten Kohlenwasserstoff-Rest mit 6 bis 200 C-Atomen optional umfassend mindestens ein Heteroatom O oder N, einen bivalenten Rest umfassend Aryl-, Arylalkyl-Gruppen optional umfassend mindestens ein Heteroatom O oder N oder olefinischen Polyether.

21. Verfahren nach Anspruch 17,
**dadurch gekennzeichnet,**
**dass** ein Polysiloxan-Diisocyanat der Formel VII,
OCN-Q2"-D-(O=C)-A-Q2'-B-Q1'-A-(C=O)-D-Q1 "-NCO (VII)
mit B einem linearen und/oder verzweigten Polysiloxan-Polymerblock B, mit -Q2'-und -Q1'- jeweils unabhängig umfassend einen bivalenten Kohlenwasserstoff-Rest mit 6 bis 200 C-Atomen optional umfassend mindestens ein Heteroatom O, N oder S, einen bivalenten Rest umfassend Aryl-, Arylalkyl-Gruppen optional umfassend mindestens ein Heteroatom O, N oder S, oder Alkyl-, Aryl- oder Alkyl- und Aryl-Gruppen enthaltende Polyether-Reste, mit A jeweils unabhängig gleich -NH-, -O-oder -S- und D gleich -NH- jeweils unabhängig in Formel VII, und mit -Q2"-und/oder -Q1"- unabhängig ausgewählt aus einem bivalenten, linearen, verzweigten und/oder cyclischen Alkyl-Rest mit 4 bis 200 C-Atomen, oder einem bivalenten Rest umfassend einen Aryl- und/oder Arylalkyl-Rest mit 6 bis 200 C-Atomen, umgesetzt wird mit einem Hydroxy- oder Amino-funktionellen UV/Vis-Chromophor, insbesondere einem Hydroxy-funktionellen UV-Chromophor, bevorzugt einem Chromophor mit mindestens einem Absorptionsmaximum von 300 bis 380 nm.

22. Verfahren nach Anspruch 17 oder 21,
**dadurch gekennzeichnet,**
**dass** das Chromophor ein Hydroxycumarin oder Hydroxycumarin-Derivat ist, insbesondere 7-Hydroxycumarin, 4-Hydroxycumarin, 6-Hydroxycumarin oder Hydroxy-Isocumarin.

23. Verfahren nach Anspruch 17,
**dadurch gekennzeichnet,**
**dass** ein Diisocyanat mit einem Hydroxy-Cumarin oder Hydroxy-Cumarin-Derivat oder dessen Salz zu einem Cumarin-Isocyanat umgesetzt wird, insbesondere zu einem Cumarin-Isocyanat ausgewählt aus den Formeln IXa und IXb, wie vorstehend definiert.

24. Zusammensetzung erhältlich nach einem Verfahren nach einem der Ansprüche 17 bis 23.

25. Zusammensetzungen enthaltend Siloxan-Polymere nach einem der Ansprüche 1 bis 16 sowie Mischungen dieser umfassend
a) Siloxan-Polymere der allgemeinen Formel XI sowie Mischungen dieser oder
b) Siloxan-Polymere mit einem zentralen Polysiloxan-Polymerblock B ausgewählt
aus (i) mindestens ein Siloxan-Polymer der allgemeinen Formel I sowie Mischungen umfassend dieses Polymer,
(ii) mindestens ein Siloxan-Polymer der allgemeinen Formel Ia sowie Mischungen umfassend dieses Polymer oder
(iii) mindestens ein Siloxan-Polymer der allgemeinen Formel Ib sowie Mischungen umfassend dieses Polymer.

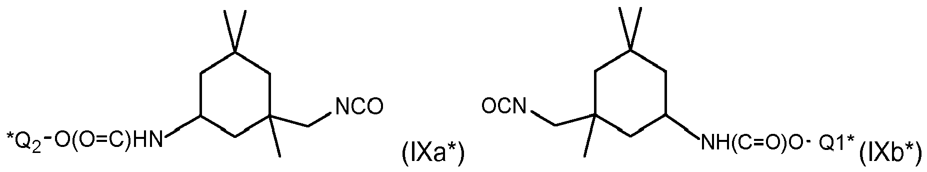

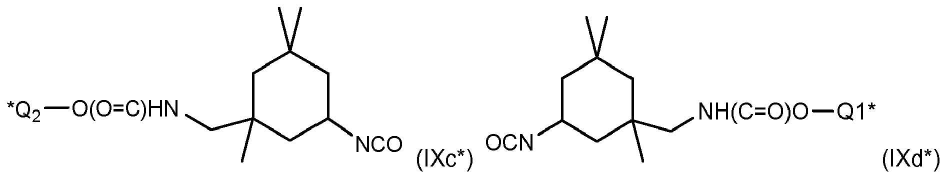

26. Zwischenprodukt zur Herstellung von Siloxan-Polymeren der Formel I nach einem der Ansprüche 1 bis 16, ausgewählt aus Cumarin-Isocyanaten ausgewählt aus den Formeln IXa, IXb, IXc und IXd
deren Salzen oder Mischungen davon
Q2*-O(CO)NH-"2Q-NCO (IXa) und/oder Q1*-O(CO)NH-"1Q-NCO (IXb)
Q2*-O(CO)NH-Q2"-NCO (IXc) und/oder Q1*-O(CO)NH-Q1"-NCO (IXd)
mit Q2*, Q2", Q1* und Q1", wie vorstehend definiert, wobei Q2* und Q1* jeweils unabhängig Cumarin oder ein Cumarin-Derivat sind, vorzugsweise Cumarin-Isocyanate der Formeln IXa*, IXb*, IXc*, IXd* oder deren Salze,

27. Formulierung enthaltend mindestens ein Siloxan-Polymere nach einem der Ansprüche 1 bis 16, 24, 25 oder hergestellt nach einem der Ansprüche 17 bis 23 oder hergestellt über das Zwischenprodukt nach Anspruch 26 und mindestens einen Hilfsstoff.

28. Verwendung der Siloxan-Polymere nach einem der Ansprüche 1 bis 16 oder der erhaltenen Zusammensetzungen umfassend Siloxan-Polymere nach einem der Ansprüche 17 bis 23 oder der Zusammensetzungen nach Anspruch 24 oder 25 als Additiv in kosmetischen Formulierungen, als Additiv in pharmazeutischen Formulierungen, in Lacken, Pasten, als Schaumstabilisator oder Schaumadditiv für Polyurethanschäume, insbesondere Polyurethanhartschäume und Polyurethanweichschäume, als Griffverbessere oder Imprägniermittel bei der bei der Herstellung von Fasern, Textilien, in kosmetischen Formulierungen zur Behandlung, Nachbehandlung und Schutz von keratinischen Fasern sowie Haut und Hautanhangsgebilden, als Additiv in Waschmittel-, Weichspülerformulierungen, in kosmetischen Formulierungen umfassend Cremes, Spülungen, Haarwachsmittel, Waschmittel, Festiger, Pflegespülungen, pflegende Pasten, Sprays, Haarsprays, zur Verbesserung der Kämmbarkeit von keratinischen oder textilen Fasern natürlichen oder synthetischen Ursprungs.
